# EUROPEAN PATENT APPLICATION

(11) **EP 1 932 831 A1**
(43) Date of publication of application: **18.06.2008**
(21) Application number: 06090223.6
(22) Date of filing: 13.12.2006
(51) Int. Cl.: C07D 209/20, C07D 401/12, C07D 403/12, C07D 409/12, A61K 31/405, A61P 15/08, C07D 405/12

(54) **1,2-Diarylacetylene Derivatives of Acyltryptophanols**

(71) Applicant: Bayer Schering Pharma Aktiengesellschaft, 13353 Berlin (DE)
(72) Inventor: Wortmann, Lars, 13353 Berlin (DE); Muhn, Hans-Peter, 13353 Berlin (DE); Menzenbach, Bernd, 07743 Jena (DE); Schrey, Anna, Institute of, 13125 Berlin (DE); Kühne, Ronald, Institute of, 13125 Berlin (DE); Kosemund, Dirk, 13353 Berlin (DE); Koppitz, Marcus, 13353 Berlin (DE)

(57) **Abstract**

The present invention relates to acyltryptophanols of the general formula I, 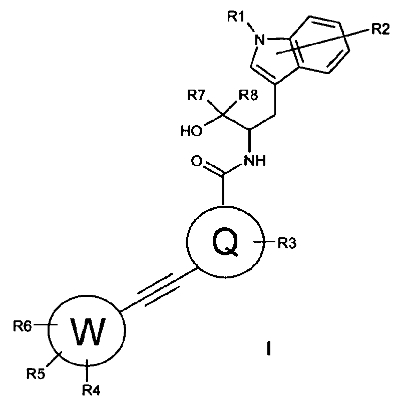 in which Q, W, R1, R2, R3, R4, R5, R6, R7 and R8 have the meaning as defined in the description.

The compounds according to the invention are effective FSH antagonists and can be used for example for fertility control in men or in women, or for the prevention and/or treatment of osteoporosis.

## Description

The present invention relates to novel 1 ,2-diarylacetylene substituted acyltryptophanols, process for their preparation, pharmaceutical compositions comprising the compounds according to the invention, and the use thereof for fertility control in men or in women as well as treatment and prevention of osteoporosis.
Follicle-stimulating hormone (FSH) and luteinizing hormone (LH) are together responsible for the control of male and female fertility and of the production of sex steroids.
In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.
In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.
FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).
The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled man expects advantages from non-peptidergic FSH agonists when used to promote fertility in women (stimulation of follicle ripening). There are no reports of experience on the use of FSH or FSH agonists in male infertility, but specific indications are also conceivable in this connection.
New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).
FSH receptors have been detected in osteoclasts but not in osteoblasts. In vitro, FSH stimulates bone resorption by mouse osteoclasts ( Li Sun et al. Cell 2006; 125: 247-60). A clinical correlation between the height of the serum FSH levels and low bone density has been observed in postmenopausal women (Devleta et al, J. Bone Miner. Metab. 2004, 22: 360-4).
These findings among others suggest that FSH stimulates loss of bone mass, and accordingly FSH antagonists will display an antiresorptive effect on bone and are therefore suitable for the therapy and/or prevention of peri- and postmenopausal loss of bone mass and osteoporosis.
FSH receptor modulators, i.e. FSH receptor antagonists and FSH receptor agonists of various compound classes of low molecular weight have been reported on recently. FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides].
FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370, WO 06/117371, [hexahydroquinolines].
FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645 [pyrrolobenzodiazepines] and PCT/EP2006/00794 [broad class of acyltryptohanols, as prior right only].

In view of the prior art, the objective technical problem to be solved according to the present invention may therefore be seen in providing alternative compounds having an FSH receptor antagonistic effect.
The technical problem has been solved according to the present invention by the provision of novel compounds of the formula I 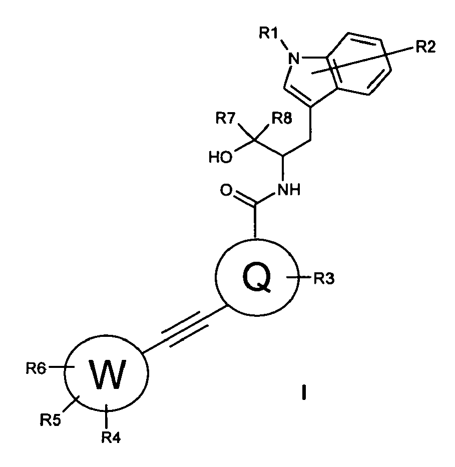 in which
- R1: is hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene; 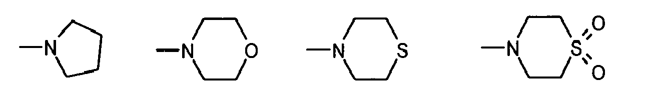 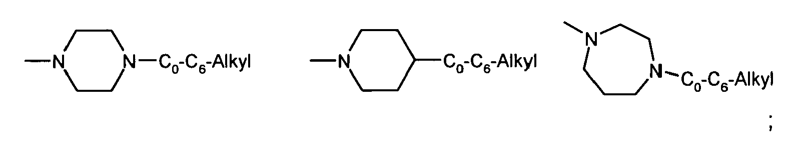
- R2: is hydrogen, halogen, cyano, -SO₂Me, C₁-C₆-alkyl, C₂-C₆-alkenyl, C₂-C₆-alkynyl, C₁-C₆-alkyloxy or benzyloxy,
where the hydrocarbon chains therein may optionally be fluorinated once or more times;
- R3: is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted once or more times by fluorine, cyano, hydroxy, amino or the groups: 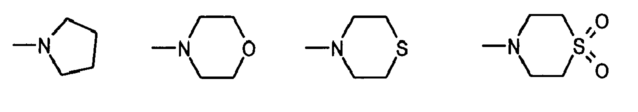 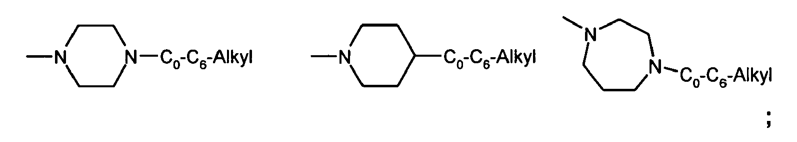
- R4, R5, R6: are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the groups: 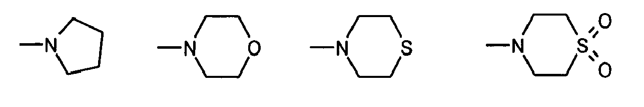 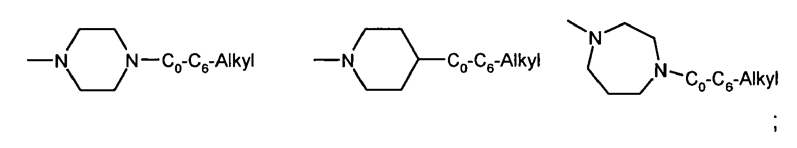 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl, C₁-C₆-alkylcarbonyl, C₃-C₇-cycloalkylcarbonyl,
carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)-alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)-amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the groups: 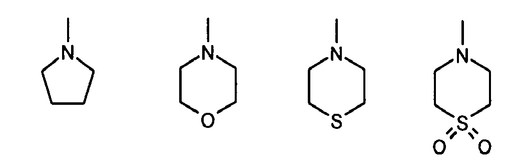 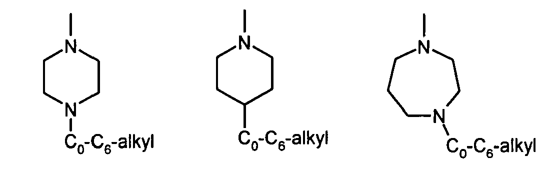 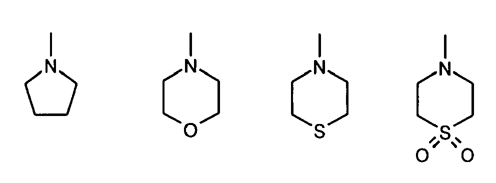 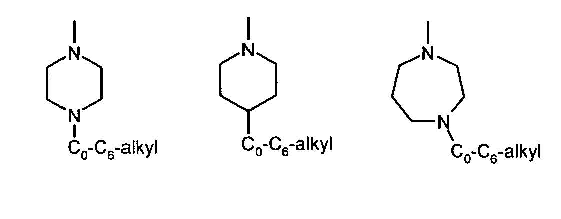 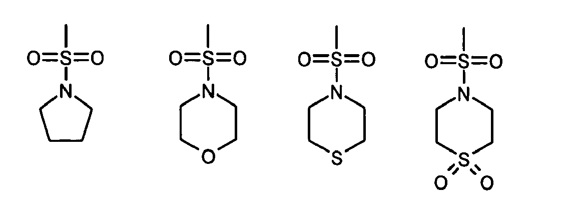 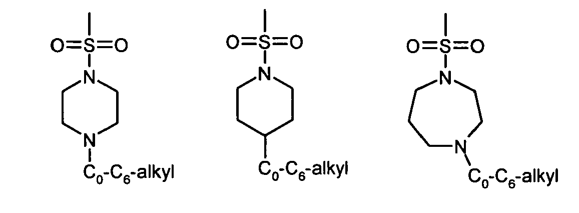 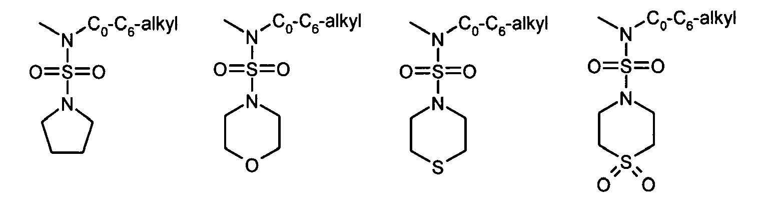 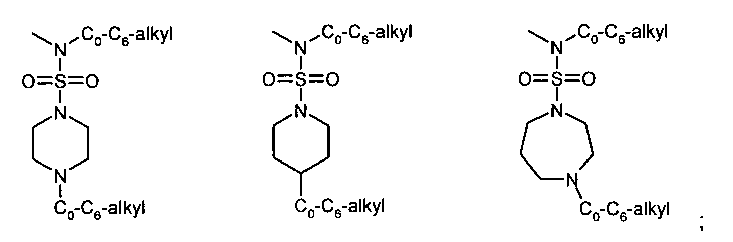
- R7, R8: are independently of one another hydrogen, methyl, ethyl, where the methyl and ethyl groups may be fluorinated once or more times;
wherein
- R2: substitutes one or more positions of the aryl or heteroaryl ring in the indole residue;
- R3: substitutes one or more positions of the aryl or heteroaryl ring in the group Q;
- R5 and R6: may together form a heterocycloalkyl or cycloalkyl group;
- Q and W: are independently of one another aryl, heteroaryl.

The present invention relates to both possible enantiomers of compounds of formula I with respect to the chirality centre in the tryptophanol moiety.

The unbranched C₁-C₆-alkyl groups for the radicals R1 to R6 may be for example a methyl, ethyl, propyl, butyl, pentyl or a hexyl group; and the branched C₃-C₆-alkyl groups for the radicals R1 to R6 may be an *iso*propyl, *iso*butyl, *sec*-butyl, *tert*-butyl, *iso*pentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.
The branched or unbranched C₃-C₆-alkenyl groups for the radical R1 may be for example an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-l-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (Z)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl- or a 1-(1,1-dimethylethyl)ethenyl group.

The C₃-C₆-alkynyl groups for the radical R1 may be for example a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₂-C₆-alkenyl groups for the radicals R2 to R6 may, in addition to the C₃-C₆-alkenyl groups mentioned for the radical R1, be for example a vinyl group.

The C₂-C₆-alkynyl groups for the radicals R2 to R6 may, in addition to the C₃-C₆-alkynyl groups mentioned for the radical R1, be for example an ethynyl group.
The C₁-C₆-alkyloxy groups for the radicals R2 to R6 may be for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R2 to R6 are fluorine, chlorine, bromine or iodine.

The C₁-C₃-alkylsulphanyl groups for the radicals R4 to R6 may be for example a methylsulphanyl (CH₃S-), ethylsulphanyl (CH₃CH₂S-), propylsulphanyl, *iso*propylsulphanyl group.

The C₁-C₆-alkylaminocarbonyl groups for the radicals R4 to R6 may be for example a methylaminocarbonyl-, ethylaminocarbonyl-, propylaminocarbonyl-, *iso*propylaminocarbonyl-, butylaminocarbonyl-, *iso*butylaminocarbonyl-, *sec*-butylaminocarbonyl-, *tert-*butylaminocarbonyl-, pentylaminocarbonyl-, *iso*pentylaminocarbonyl-, (2-methylbutyl)-aminocarbonyl-, (1-methylbutyl)aminocarbonyl-, (1-ethylpropyl)aminocarbonyl-, *neo-penty*laminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.
The hydroxy-C₁-C₆-alkylene groups for the radicals R3 to R6 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may be formed by the groups R5 and R6 together include for example the following (biradical) groups:

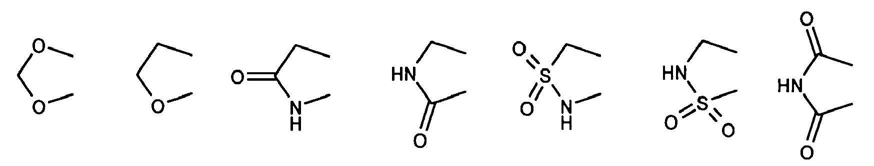

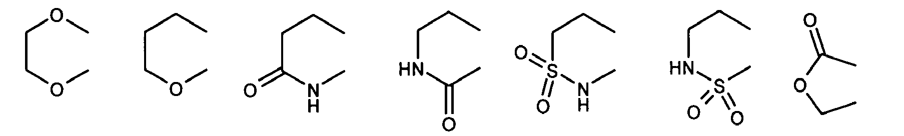

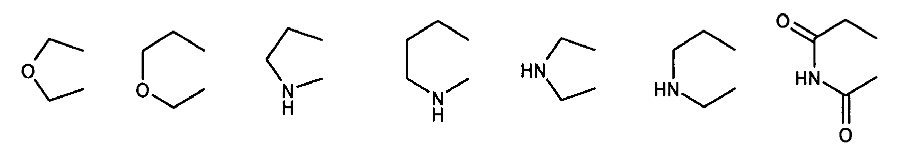

The cycloalkyl groups which may be formed by the the groups R5 and R6 together include for example the following (biradical) groups:

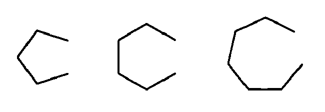

The C₃-C₇-cycloalkyl groups for the radicals R1 to R6 may be for example a cyclopropyl, cyclobutyl, cyclopentyl, cyclohexyl, cycloheptyl group.
The C₃-C₇-heterocycloalkyl groups for the radicals R1 to R6 may be for example a cyclopropyl, cyclobutyl, cycopentyl, cyclohexyl, cycloheptyl group in which one or two carbon atoms of the ring are replaced independently of one another by an oxygen, nitrogen or sulphur atom.
The aryl groups for the radicals Q and W may be for example a phenyl, naphthyl group which is linked via substitutable positions.
The heteroaryl groups for the radicals Q and W may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.
The C₃-C₇-cycloalkyloxy groups for the radicals R1 to R6 may be for example a cyclopropyloxy, cyclobutyloxy, cyclopentyloxy, cyclohexyloxy, cycloheptyloxy group.
The C₁-C₆-alkylamino groups for the radicals R1 to R6 may be for example methylamino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neo*pentylamino, (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.
In the di(C₁-C₆-alkyl)amino groups for the radicals R1 to R6, each of the two radicals on the nitrogen atom of the dialkylamino group may be chosen independently of one another from the following radicals: possible examples are a methyl, ethyl, propyl, isopropyl, butyl, *iso*butyl, *sec*-butyl, *tert*-butyl, pentyl, *iso*pentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the C₃-C₇-cycloalkyl-C₁-C₆-alkyleneoxy groups for the radicals R1 to R6 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C₀-C₆-alkyleneoxy group, for example with a methyleneoxy, ethyleneoxy, propyleneoxy, butyleneoxy, pentyleneoxy, hexyleneoxy group.
In the hydroxy-C₃-C₆-alkenylene groups for the radicals R1 to R6 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group, and to be combined independently of one another.
In the hydroxy-C₃-C₆-alkynyl groups for the radicals R1 to R6 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.
In the C₁-C₆-alkyloxy-C₃-C₆-alkenylene groups for the radicals R1 to R6 it is possible for the C₁-C₆-alkyloxy group, for example a methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another. In the C₁-C₆-alkyloxy-C₃-C₆-alkynylene groups for the radicals R1 to R6 it is possible for the C₁-C₆-alkyloxy group, for example a methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy,
(2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group, and to be combined independently of one another.
In the C₁-C₆-alkyloxyphenyl-C₁-C₆-alkylene groups for the radical R1 to R6 it is possible for the C₁-C₆-alkyloxy group to be selected independently of one another from methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy, and to be combined independently of one another with C₁-C₆-alkylene groups such as, for example, methylene, ethylene, propylene, butylene, pentylene, hexylene.
In the C₃-C₇-cycloalkyl-(C₀-C₆)-alkyleneamino groups of the radicals R3 to R6 it is possible for each of the C₃-C₇-cycloalkyl groups of the C₃-C₇-cycloalkyl-(C₀-C₆)-alkyleneamino group, for example of a cyclopropyl, cyclobutyl, cyclopentyl, cyclohexyl or cycloheptyl group, to be combined independently of one another with each C₀-C₆-alkylene group, for example with a bond, a methylene, ethylene, propylene, butylene, pentylene, hexylene group.
In the C₁-C₆-alkyloxy-C₁-C₆-alkylene groups for the radical R1 to R6, it is possible for the C₁-C₆-alkyloxy group to be selected independently for example from methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy,
(2,3-dimethylbutyl)oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.
In the di(C₁-C₆-alkyl)amino-C₁-C₆-alkylene group for the radical R1 it is possible for each of the two radicals on the nitrogen atom of the amino group to be selected independently for example from methyl, ethyl, propyl, isopropyl, butyl, isobutyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.
The C₃-C₇-cycloalkyl-C₁-C₆-alkylene groups for the radicals R1 to R6 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group. In the C₁-C₆-alkylamino-C₁-C₆-alkylene groups for the radicals R1 to R6 it is possible for the C₁-C₆-alkylamino group to be selected independently for example from methylamino, ethylamino, propylamino, *iso*propylamino, butylamino, *iso*butylamino, *sec*-butylamino, *tert*-butylamino, pentylamino, isopentylamino, (2-methylbutyl)amino, (1-methylbutyl)amino, (1-ethylpropyl)amino, *neo*pentylamino, (1,1-dimethylpropyl)amino, hexylamino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.
The phenyloxy-C₁-C₆-alkylene groups for the radicals R1 to R6 may be for example a phenyloxymethyl, phenyloxyethyl, phenyloxypropyl, phenyloxybutyl, phenyloxypentyl, phenyloxyhexyl group.
In the C₁-C₆-acyl-(C₀-C₆-alkyl)amido groups for the radicals R4 to R6, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethylbutyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)amido, (2-ethylbutyl)amido, (3,3-dimethylbutyl)amido, (2,2-dimethylbutyl)-amido, (1,1-dimethylbutyl)amido, (2,3-dimethylbutyl)amido, (1,3-dimethylbutyl)amido or a (1,2-dimethylbutyl)amido group.
The C₁-C₆-alkylaminocarbonyl groups for the radicals R4 to R6 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)aminocarbonyl, (1,1-dimethylbutyl)aminocarbonyl, (2,3-dimethylbutyl)aminocarbonyl, (1,3-dimethylbutyl)-aminocarbonyl or a (1,2-dimethylbutyl)aminocarbonyl group.
In the di(C₁-C₆-alkyl)aminocarbonyl groups for the radicals R4 to R6, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
The (C₃-C₇-cycloalkyl)aminocarbonyl groups for the radicals R4 to R6 may be for example a cyclopropylaminocarbonyl, cyclobutylaminocarbonyl, cyclopentylaminocarbonyl, cyclohexylaminocarbonyl or cycloheptylaminocarbonyl group.
In the di(C₃-C₇-cycloalkyl)aminocarbonyl groups for the radicals R4 to R6, each of the two C₃-C₇-cycloalkyl radicals on the nitrogen atom of the di(C₃-C₇-cycloalkyl)aminocarbonyl group may be independently of one another for example a cyclopropyl, cyclobutyl, cyclopentyl, cyclohexyl or cycloheptyl group.
In the C₃-C₇-cycloalkyl-C₁-C₆-alkyleneaminocarbonyl groups of the radicals R4 to R6 it is possible for each of the C₃-C₇-cycloalkyl groups of the C₃-C₇-cycloalkyl-C₁-C₆-alkyleneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyleneaminocarbonyl, propyleneaminocarbonyl, butyleneaminocarbonyl, pentyleneaminocarbonyl, hexyleneaminocarbonyl group.
The C₁-C₆-alkylcarbonyl groups for the radicals R4 to R6 may be for example a methylcarbonyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)-carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)carbonyl or a (1,2-dimethylbutyl)carbonyl group.
The C₃-C₇-cycloakylcarbonyl groups for the radicals R4 to R6 may be for example a cyclopropylcarbonyl, cyclobutylcarbonyl, cyclopentylcarbonyl, cyclohexylcarbonyl or cycloheptylcarbonyl group.
The C₁-C₆-alkyloxycarbonyl groups for the radicals R4 to R6 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R4 to R6 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)sulphonyl, (3-methylpentyl)sulphonyl, (2-methylpentyl)sulphonyl, (1-methylpentyl)sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1,3-dimethylbutyl)sulphonyl or a (1,2-dimethylbutyl)sulphonyl group.
The C₃-C₇-cycloalkylsulphonyl groups for the radicals R4 to R6 may be for example a cyclopropylsulphonyl, cyclobutylsulphonyl, cyclopentylsulphonyl, cyclohexylsulphonyl or cycloheptylsulphonyl group.
In the C₃-C₇-cycloalkyl-C₁-C₆-alkylenesulphonyl groups of the radicals R4 to R6 it is possible for each of the C₃-C₇-cycloalkyl groups of the C₃-C₇-cycloalkyl-C₁-C₆-alkylenesulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group. The C₁-C₆-alkylaminosulphonyl groups for the radicals R4 to R6 may be for example a methylaminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)-aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)aminosulphonyl, (1-methylpentyl)aminosulphonyl, (1-ethylbutyl)aminosulphonyl, (2-ethylbutyl)-aminosulphonyl, (3,3-dimethylbutyl)aminosulphonyl, (2,2-dimethylbutyl)aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.
In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
The (C₃-C₇-cycloalkyl)aminosulphonyl groups for the radicals R4 to R6 may be for example a cyclopropylaminosulphonyl, cyclobutylaminosulphonyl, cyclopentylaminosulphonyl, cyclohexylaminosulphonyl or cycloheptylaminosulphonyl group.
In the di(C₃-C₇-cycloalkyl)aminosulphonyl groups for the radicals R4 to R6, each of the two C₃-C₇-cycloalkyl radicals on the nitrogen atom of the di(C₃-C₇-cycloalkyl)aminosulphonyl group may be independently of one another for example a cyclopropyl, cyclobutyl, cyclopentyl, cyclohexyl or cycloheptyl group.
In the C₃-C₇-cycloalkyl-C₁-C₆-alkyleneaminosulphonyl groups of the radicals R4 to R6, each of the C₃-C₇-cycloalkyl groups of the C₃-C₇-cycloalkyl-C₁-C₆-alkyleneaminosulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.
The C₁-C₆-alkylsulphonylamido groups for the radicals R4 to R6 may be for example a methylsulphonylamido, ethylsulphonylamido, propylsulphonylamido, isopropylsulphonylamido, butylsulphonylamido, isobutylsulphonylamido, sec-butylsulphonylamido, tertbutylsulphonylamido, pentylsulphonylamido, isopentylsulphonylamido, (2-methylbutyl)-sulphonylamido, (1-methylbutyl)sulphonylamido, (1-ethylpropyl)sulphonylamido, neopentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.
In the -N(C₀-C₆-alkyl)-C(O)-C₁-C₆-alkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N-(C₀-C₆-alkyl)-C(O)-C₃-C₇-cycloalkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.
In the -N(C₀-C₆-alkyl)-C(O)-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R4 to R6, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-C(O)-NH-(C₃-C₇-cycloalkyl) groups of the radicals R4 to R6, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl),
(1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.
In the -N(C₀-C₆-alkyl)-SO₂-(C₁-C₆-alkyl) groups of the radicals R4 to R6, each of the (C_{O}-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-SO₂-C₃-C₇-cycloalkyl groups of the radicals R4 to R6, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.
In the -N(C₀-C₆-alkyl)-SO₂-N-di(C₀-C₆-alkyl) groups of the radicals R4 to R6, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-SO₂-NH-(C₃-C₇)-cycloalkyl groups of the radicals R4 to R6, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.
In the -C(O)-N(H)-C₂-C₆-alkylene-(C₁-C₆-alkyl)amine groups of the radicals R4 to R6, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -C(O)-N(H)-C₂-C₆-alkylene-[di(C₁-C₆-alkyl)]amine groups of the radicals R4 to R6, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -C(O)-N(H)-C₂-C₆-alkylene-(C₃-C₇-cycloalkyl)amine groups of the radicals R4 to R6, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.
In the -C(O)-N(H)-C₂-C₆-alkylene-(C₃-C₇-cycloalkyl-C₁-C₆-alkylene)amine groups of the radicals R4 to R6, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R4 to R6, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -S(O₂)-N(H)-C₂-C₆-alkylene-[di(C₁-C₆-alkyl)]amine groups of the radicals R4 to R6, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -S(O₂)-N(H)-C₂-C₆-alkylene-(C₃-C₇-cycloalkyl)amine groups of the radicals R4 to R6, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.
In the -S(O₂)-N(H)-C₂-C₆-alkylene-(C₃-C₇-cycloalkyl-C₁-C₆-alkylene)amine groups of the radicals R4 to R6, each C₂-C₆-alkylene group of the -S(O₂)-N(H)-C₂-C₆-alkylene-(C₃-C₇-cycloalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.
In the -O-C₂-C₆-alkylene-(C₁-C₆-alkyl)amine groups of the radicals R4 to R6, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -O-C₂-C₆-alkylene-[di(C₁-C₆-alkyl)]amine groups of the radicals R4 to R6, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are those of the formula II or III 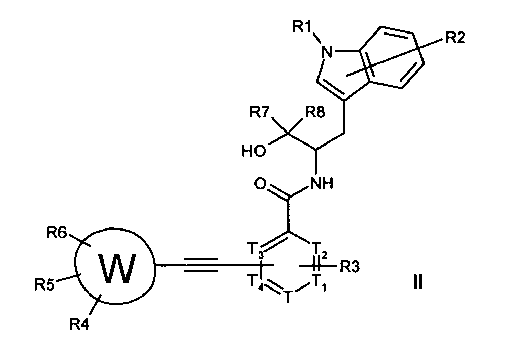 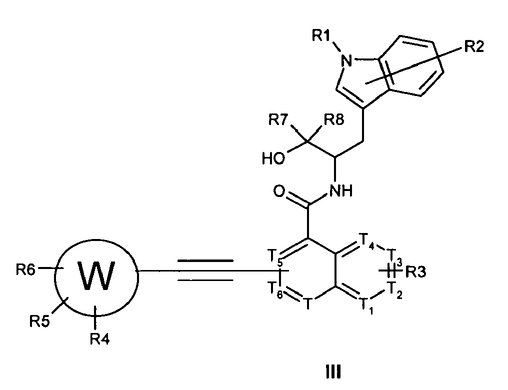 in which the groups R1 to R8 and W have the same meaning as in formula I
and
- T: is a nitrogen atom or a CH group;
- T1, T2, T3, T4, T5, T6: are each independently of one another a nitrogen atom or an R3-C group.

Compounds particularly preferred according to the present invention are those of the formula IV or V 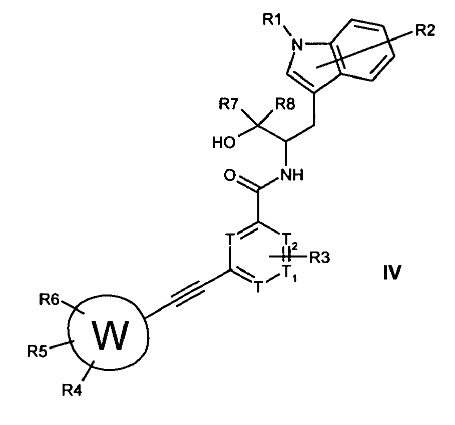 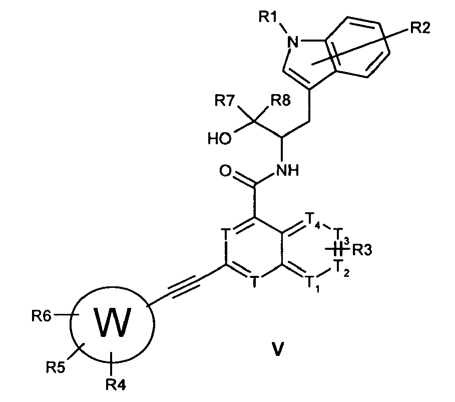 in which
- R1 to R8 and W: have the same meaning as in formula I;
- T: is a nitrogen atom or a CH group;
- T1, T2, T3, T4: are each independently of one another a nitrogen atom or an R3-C group.

The following compounds are very particularly preferred:
**1** N-[(R)-1-Hydroxymethyl-2-(1H-indol-3-yl)-ethyl]-5-phenylethynyl-2-propoxy-benzamide
**2** N-[(R)-1-Hydroxymethyl-2-(1H-indol-3-yl)-ethyl]-5-(2-methoxy-phenylethynyl)-2-propoxy-benzamide
**3** N-[(R)-1-Hydroxymethyl-2-(1H-indol-3-yl)-ethyl]-2-isopropoxy-5-(2-methoxyphenylethynyl)-benzamide
**4** N-[(S)-1-Hydroxymethyl-2-(1H-indol-3-yl)-ethyl]-2-isopropoxy-5-(2-methoxyphenylethynyl)-benzamide
**5** N-[(R)-1-Hydroxymethyl-2-(1H-indol-3-yl)-ethyl]-2-isopropoxy-5-o-tolylethynyl-benzamide
**6** 3-(2-Methoxy-phenylethynyl)-naphthalene-1-carboxylic acid [(R)-1-hydroxymethyl-2-(1H-indol-3-yl)-ethyl]-amide
**7** 6-(2-Methoxy-phenylethynyl)-quinoline-8-carboxylic acid [(R)-1-hydroxymethyl-2-(1H-indol-3-yl)-ethyl]-amide
**8** N-[(R)-1-Hydroxymethyl-2-(1H-indol-3-yl)-ethyl]-2-isopropoxy-5-(3-ethylcarbamoylphenylethynyl)-benzamide
**9** 2-Ethoxy-N-[(R)-1-hydroxymethyl-2-(1H-indol-3-yl)-ethyl]-5-(3-methylcarbamoylphenylethynyl)-benzamide
**10** N-[(R)-1-Hydroxymethyl-2-(1H-indol-3-yl)-ethyl]-2-isopropoxy-5-(4-methylcarbamoylphenylethynyl)-benzamide
**11** N-[(R)-1-Hydroxymethyl-2-(1H-indol-3-yl)-ethyl]-2-isopropoxy-5-(1-oxo-1,2,3,4-tetrahydro-isoquinolin-7-ylethynyl)-benzamide
**12** 2-Ethoxy-N-[(R)-1-hydroxymethyl-2-(1H-indol-3-yl)-ethyl]-5-(4-methylcarbamoylphenylethynyl)-benzamide
**13** 5-(2,3-Dihydro-1H-indol-5-ylethynyl)-N-[(R)-1-hydroxymethyl-2-(1H-indol-3-yl)-ethyl]-2-isopropoxy-benzamide
**14** 5-(5-Cyanomethyl-2-methoxy-phenylethynyl)-N-[(R)-1-hydroxymethyl-2-(1H-indol-3-yl)-ethyl]-2-isopropoxy-benzamide
**15** 5-(2,3-Dimethyl-phenylethynyl)-N-[(R)-1-hydroxymethyl-2-(1H-indol-3-yl)-ethyl]-2-isopropoxy-benzamide
**16** 5-(2-Cyano-thiophen-3-ylethynyl)-N-[(R)-1-hydroxymethyl-2-(1H-indol-3-yl)-ethyl]-2-isopropoxy-benzamide
**17** N-[(R)-1-Hydroxymethyl-2-(1H-indol-3-yl)-ethyl]-2-isopropoxy-5-(2,4,6-trimethylphenylethynyl)-benzamide
**18** N-[(R)-1-Hydroxymethyl-2-(1H-indol-3-yl)-ethyl]-2-isopropoxy-5-(2,4,5-trimethylphenylethynyl)-benzamide
**19** 5-[4-(2-Hydroxy-ethyl)-phenylethynyl]-N-[(R)-1-hydroxymethyl-2-(1H-indol-3-yl)-ethyl]-2-isopropoxy-benzamide
**20** N-[(R)-2-Hydroxy-1-(1H-indol-3-ylmethyl)-2-methyl-propyl]-2-isopropoxy-5-(2-methoxyphenylethynyl)-benzamide
**21** N-[2-(5-Fluoro-1H-indol-3-yl)-1-hydroxymethyl-ethyl]-2-isopropoxy-5-phenylethynylbenzamide
**22** N-[2-(5-Fluoro-1H-indol-3-yl)-1-hydroxymethyl-ethyl]-2-isopropoxy-5-(2-methoxyphenylethynyl)-benzamide
**23** N-[(RS)-2-(5,6-Difluoro-1H-indol-3-yl)-1-hydroxymethyl-ethyl]-2-isopropoxy-5-(2-methoxy-phenylethynyl)-benzamide
**24** N-[(R)-2-(1-Ethyl-1H-indol-3-yl)-1-hydroxymethyl-ethyl]-2-isopropoxy-5-(2-methoxyphenylethynyl)-benzamide
**25** N-[(R)-2-(1-Ethyl-1H-indol-3-yl)-1-hydroxymethyl-ethyl]-2-isopropoxy-5-phenylethynylbenzamide
**26** 5-(3-Amino-1H-indazol-4-ylethynyl)-N-[(R)-2-hydroxy-1-(1H-indol-3-ylmethyl)-ethyl]-2-isopropoxy-benzamide
**27** N-[(R)-1-Hydroxymethyl-2-(1H-indol-3-yl)-ethyl]-5-(4-hydroxy-phenylethynyl)-2-isopropoxy-benzamide
**28** N-[(R)-1-Hydroxymethyl-2-(1H-indol-3-yl)-ethyl]-2-isopropoxy-5-(6-methoxynaphthalen-2-ylethynyl)-benzamide
**29** N-[(R)-1-Hydroxymethyl-2-(1H-indol-3-yl)-ethyl]-2-isopropoxy-5-m-tolylethynyl-benzamide
**30** 5-(3-Fluoro-4-methoxy-phenylethynyl)-N-[(R)-2-hydroxy-1-(1H-indol-3-ylmethyl)-ethyl]-2-isopropoxy-benzamide
**31** N-[(R)-1-Hydroxymethyl-2-(1H-indol-3-yl)-ethyl]-5-(3-hydroxy-6-methyl-pyridin-2-ylethynyl)-2-isopropoxy-benzamide
**32** 4-{3-[(R)-1-Hydroxymethyl-2-(1H-indol-3-yl)-ethylcarbamoyl]-4-isopropoxyphenylethynyl}-benzoic acid methyl ester
**33** 5-{3-[(R)-1-Hydroxymethyl-2-(1H-indol-3-yl)-ethylcarbamoyl]-4-isopropoxyphenylethynyl}-nicotinic acid methyl ester
**34** 5-{3-[(R)-2-Hydroxy-1-(1H-indol-3-ylmethyl)-ethylcarbamoyl]-4-isopropoxyphenylethynyl}-thiophene-2-carboxylic acid ethyl
**35** N-[(R)-1-Hydroxymethyl-2-(H-indol-3-yl)-ethyl]-5-(6-hydroxy-naphthalen-2-ylethynyl)-2-isopropoxy-benzamide
**36** 3-{3-[(R)-1-Hydroxymethyl-2-(1H-indol-3-yl)-ethylcarbamoyl]-4-isopropoxyphenylethynyl}-benzoic acid
**37** N-[(R)-1-Hydroxymethyl-2-(1H-indol-3-yl)-ethyl]-5-(2-hydroxymethyl-phenylethynyl)-2-isopropoxy-benzamide
**38** 5-(5-Fluoro-2-methoxy-phenylethynyl)-N-[(R)-2-hydroxy-1-(1H-indol-3-ylmethyl)-ethyl]-2-isopropoxy-benzamide
**39** 5-(3-Cyano-phenylethynyl)-N-[(R)-1-hydroxymethyl-2-(1H-indol-3-yl)-ethyl]-2-isopropoxy-benzamide
**40** 5-(3,5-Dimethyl-phenylethynyl)-N-[(R)-1-hydroxymethyl-2-(1H-indol-3-yl)-ethyl]-2-isopropoxy-benzamide
**41** 5-(2,6-Difluoro-phenylethynyl)-N-[(R)-2-hydroxy-1-(1H-indol-3-ylmethyl)-ethyl]-2-isopropoxy-benzamide
**42** 5-(3,5-Difluoro-phenylethynyl)-N-[(R)-2-hydroxy-1-(1H-indol-3-ylmethyl)-ethyl]-2-isopropoxy-benzamide
**43** N-[(R)-2-Hydroxy-1-(1H-indol-3-ylmethyl)-ethyl]-2-isopropoxy-5-(3-trifluoromethylphenylethynyl)-benzamide
**44** 5-(2-Chloro-4-hydroxy-phenylethynyl)-N-[(R)-2-hydroxy-1-(1H-indol-3-ylmethyl)-ethyl]-2-isopropoxy-benzamide
**45** N-[(R)-1-Hydroxymethyl-2-(1H-indol-3-yl)-ethyl]-2-isopropoxy-5-(4-methoxyphenylethynyl)-benzamide
**46** N-[(R)-1-Hydroxymethyl-2-(1H-indol-3-yl)-ethyl]-2-isopropoxy-5-pyrimidin-5-ylethynylbenzamide
**47** N-[(R)-1-Hydroxymethyl-2-(1H-indol-3-yl)-ethyl]-5-(3-hydroxy-phenylethynyl)-2-isopropoxy-benzamide
**48** 5-(2,5-Dimethyl-phenylethynyl)-N-[(R)-1-hydroxymethyl-2-(1H-indol-3-yl)-ethyl]-2-isopropoxy-benzamide
**49** 5-(3,4-Dichloro-phenylethynyl)-N-[(R)-2-hydroxy-1-(1H-indol-3-ylmethyl)-ethyl]-2-isopropoxy-benzamide
**50** 5-Benzo[1,3]dioxol-5-ylethynyl-N-[(R)-1-hydroxymethyl-2-(1H-indol-3-yl)-ethyl]-2-isopropoxy-benzamide
**51** 5-(2-Cyano-phenylethynyl)-N-[(R)-1-hydroxymethyl-2-(1H-indol-3-yl)-ethyl]-2-isopropoxy-benzamide
**52** N-[(R)-1-Hydroxymethyl-2-(1H-indol-3-yl)-ethyl]-2-isopropoxy-5-naphthalen-2-ylethynyl-benzamide
**53** 5-(4-Fluoro-2-methyl-phenylethynyl)-N-[(R)-2-hydroxy-1-(1H-indol-3-ylmethyl)-ethyl]-2-isopropoxy-benzamide
**54** N-[(R)-1-Hydroxymethyl-2-(1H-indol-3-yl)-ethyl]-2-isopropoxy-5-naphthalen-1-ylethynyl-benzamide
**55** 5-(2,4-Dimethyl-phenylethynyl)-N-[(R)-1-hydroxymethyl-2-(1H-indol-3-yl)-ethyl]-2-isopropoxy-benzamide
**56** 5-(2-Fluoro-phenylethynyl)-N-[(R)-2-hydroxy-1-(1H-indol-3-ylmethyl)-ethyl]-2-isopropoxy-benzamide
**57** N-[(R)-1-Hydroxymethyl-2-(1H-indol-3-yl)-ethyl]-2-isopropoxy-5-(3-methoxyphenylethynyl)-benzamide
**58** (2-{3-[(R)-1-Hydroxymethyl-2-(1H-indol-3-yl)-ethylcarbamoyl]-4-isopropoxyphenylethynyl}-phenyl)-acetic acid
**59** 5-(4-Hydroxy-biphenyl-3-ylethynyl)-N-[(R)-1-hydroxymethyl-2-(1H-indol-3-yl)-ethyl]-2-isopropoxy-benzamide
**60** 5-(3-Chloro-4-cyano-phenylethynyl)-N-[(R)-2-hydroxy-1-(1H-indol-3-ylmethyl)-ethyl]-2-isopropoxy-benzamide
**61** 5-(3-Acetyl-phenylethynyl)-N-[(R)-1-hydroxymethyl-2-(1H-indol-3-yl)-ethyl]-2-isopropoxy-benzamide
**62** 5-(3,5-Dichloro-phenylethynyl)-N-[(R)-2-hydroxy-1-(1H-indol-3-ylmethyl)-ethyl]-2-isopropoxy-benzamide
**63** 5-(2-Acetyl-benzofuran-5-ylethynyl)-N-[(R)-1-hydroxymethyl-2-(1H-indol-3-yl)-ethyl]-2-isopropoxy-benzamide
**64** 3-Hydroxy-4-{3-[(R)-1-hydroxymethyl-2-(1H-indol-3-yl)-ethylcarbamoyl]-4-isopropoxyphenylethynyl}-benzoic acid
**65** 4-Hydroxy-3-{3-[(R)-1-hydroxymethyl-2-(1H-indol-3-yl)-ethylcarbamoyl]-4-isopropoxyphenylethynyl}-benzoic acid methyl ester

The present invention also relates to a process for preparing the compounds according to the invention. Compounds of the general formula I can be prepared as shown in Scheme 1 by an amide forming reaction between the tryptophanol derivative VI and the carboxylic acid VII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (N-[3-(dimethylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula I. Suitable reagents for preparing the intermediate carbonyl chloride are for example thionyl chloride, oxalyl chloride, phosgene or derivatives thereof.

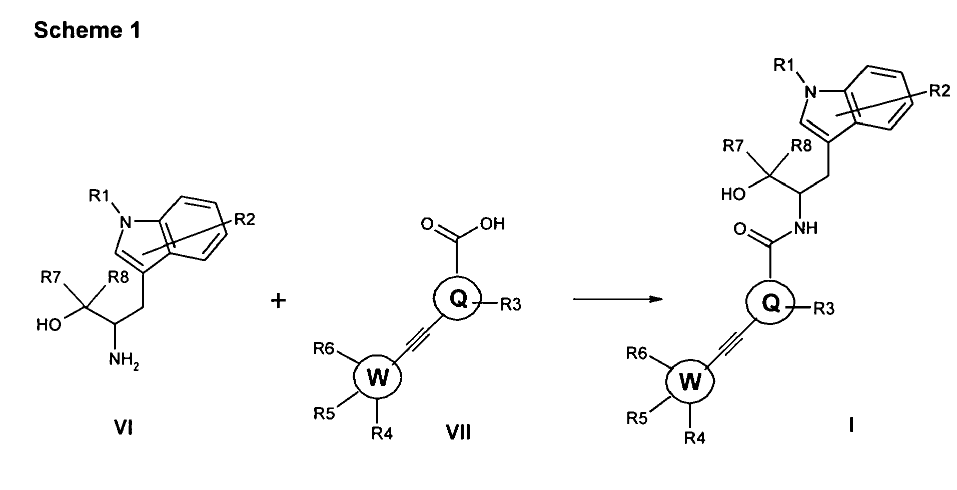

Compounds of general formulae II, III can be prepared as shown in Scheme 2 by an amide forming reaction between the tryptophanol derivative VI and the appropriate carboxylic acid VIII or IX. Reagents suitable for this purpose are all known peptide-coupling reagents which convert the carboxylic acid, where appropriate in the presence of a base, into an intermediate active ester, for example PyBOP ([(1H-benzotriazol-1-yl)oxy]tris(pyrrolidin-1-yl)phosphonium hexafluorophosphate), HATU (2-(7-aza-1*H-*benzotriazol-1-yl)-1,1,3,3-tetramethyluronium hexafluorophosphate), HBTU (2-(1*H-*benzotriazol-1-yl)-1,1,3,3-tetramethyluronium hexafluorophosphate), EDC (N-[3-(dimethylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula II or III. Suitable reagents for preparing the intermediate carbonyl chloride are for example thionyl chloride, oxalyl chloride, phosgene or derivatives thereof.

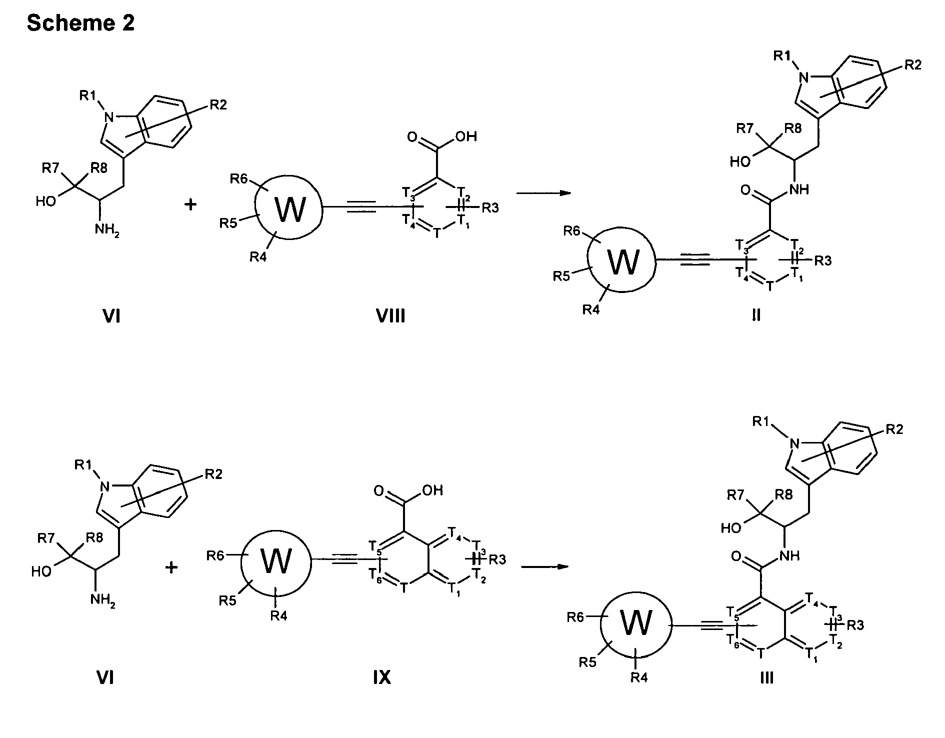

Compounds of general formulae IV and V can be prepared as shown in Scheme 3 by an amide forming reaction between the tryptophanol derivative VI and the appropriate carboxylic acid X or XI. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1H-benzotriazol-1-yl)-1,1,3,3-tetramethyluronium hexafluorophosphate), EDC (*N*-[3-(dimethylamino)propyl]-*N'*-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula IV or V. Suitable reagents for preparing the intermediate carbonyl chloride are for example thionyl chloride, oxalyl chloride, phosgene or derivatives thereof.

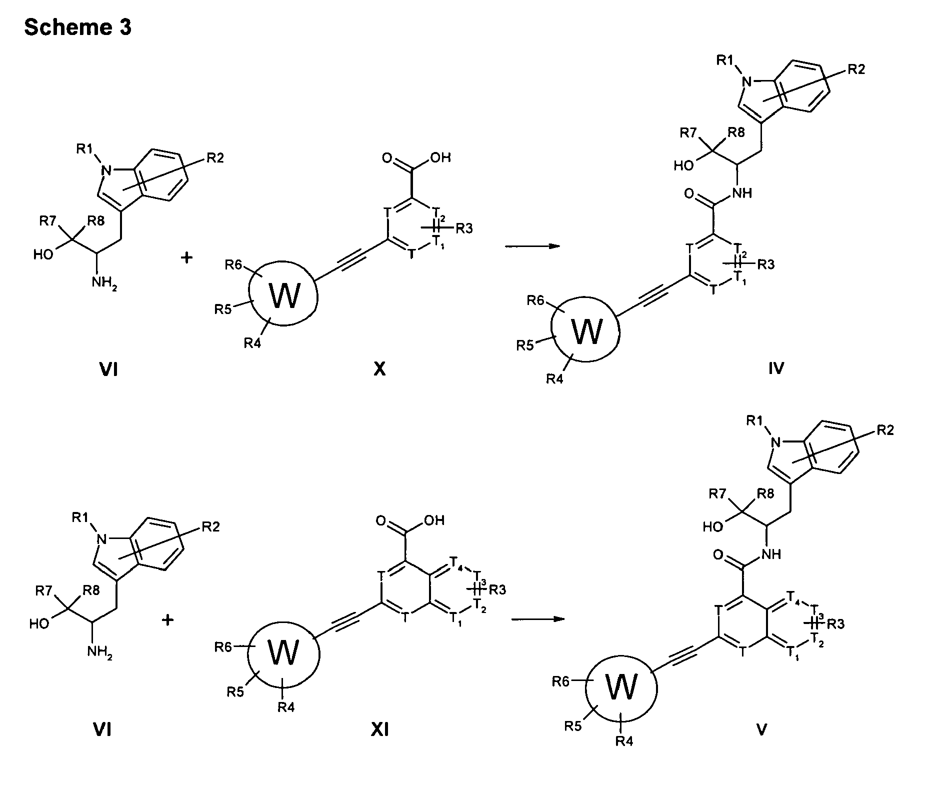

Alternatively, compounds of the general formula I can also be prepared as shown in Scheme 4 by a Sonogashira type coupling reaction between an acetylene derivative XIII or XIV and an aryl halide XII or XV. Reagents suitable for this purpose are palladium catalysts in combination with or without a Cu (I) source , with or without a base in a suitable solvent which are known to the skilled person.
For a literature review on the Sonogashira reaction, see *e.g.* S. Takahashi, Y. Kuroyama, K. Sonogashira and N. Hagihara, Synthesis, 1980, 627; K. Sonogashira, in Metal catalyzed Cross-coupling Reactions, ed. F. Diederich and P. J. Stang, Wiley-VCH, Weinheim, 1998, p. 203; K. Sonogashira, J. Organomet. Chem., 2002, 653 (1-2), 46.

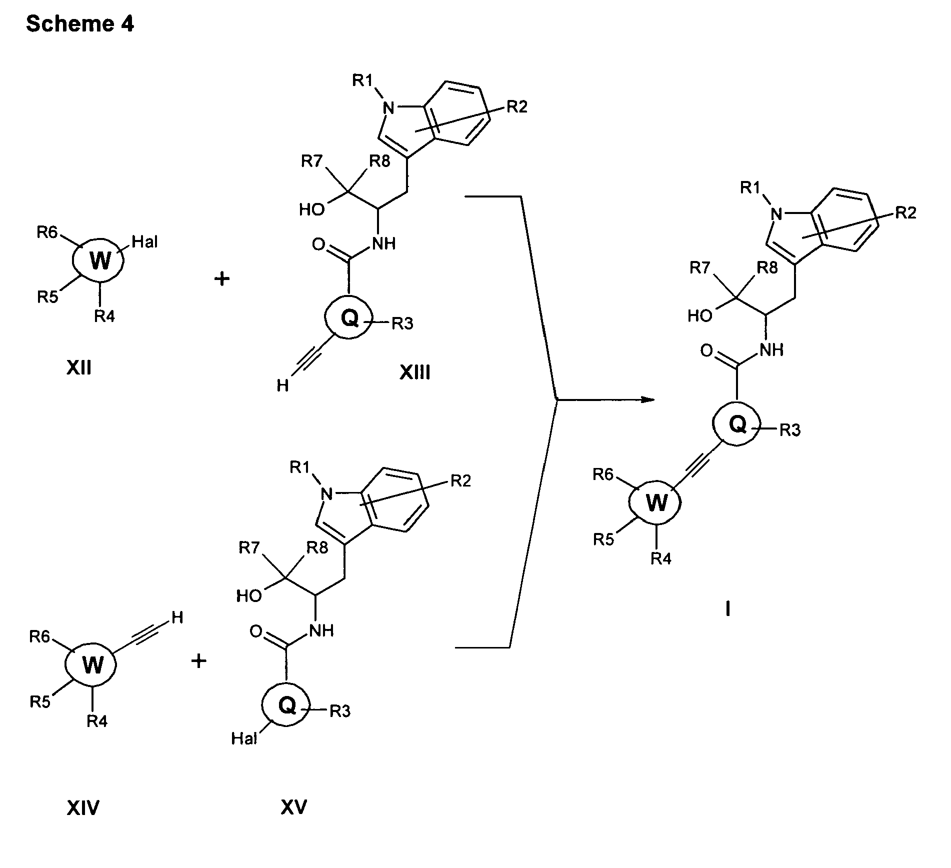

Likewise, compounds of the general formula II can be prepared as shown in Scheme 5 by a Sonogashira type coupling reaction between an acetylene derivative XVI or XIV and an aryl halide XII or XVII. Reagents suitable for this purpose are palladium catalysts in combination with or without a Cu (I) source , with or without a base in a suitable solvent which are known to the skilled person.

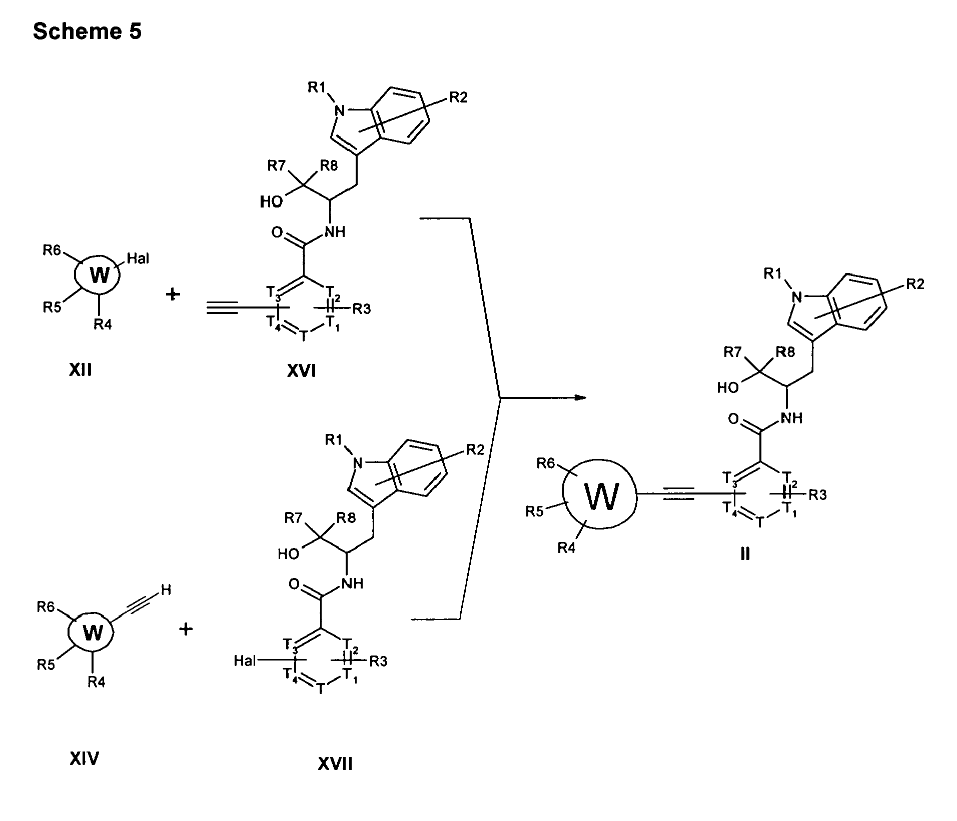

Likewise, compounds of the general formula III can be prepared as shown in Scheme 6 by a Sonogashira type coupling reaction between an acetylene derivative XVIII or XIV and an aryl halide XII or XIX. Reagents suitable for this purpose are palladium catalysts in combination with or without a Cu (I) source , with or without a base in a suitable solvent which are known to the skilled person.

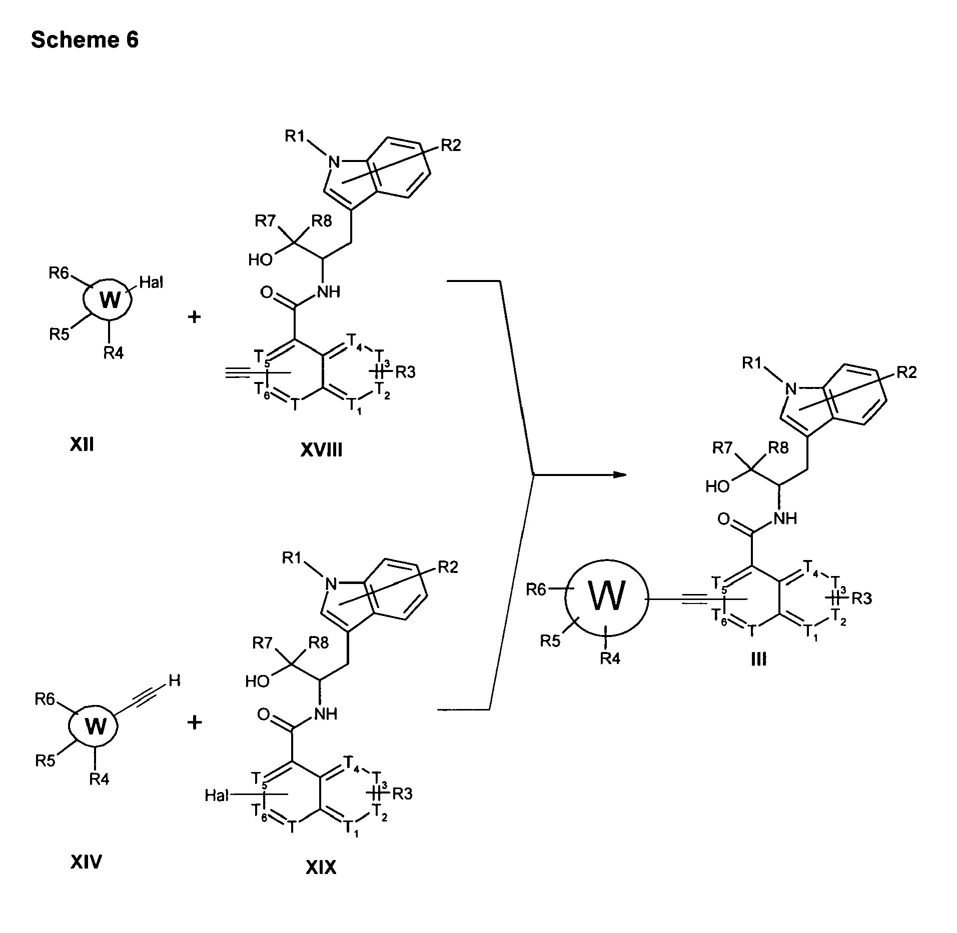

Likewise, compounds of the general formula IV can be prepared as shown in Scheme 7 by a Sonogashira type coupling reaction between an acetylene derivative XX or XIV and an aryl halide XII or XXI. Reagents suitable for this purpose are palladium catalysts in combination with or without a Cu (I) source , with or without a base in a suitable solvent which are known to the skilled person.

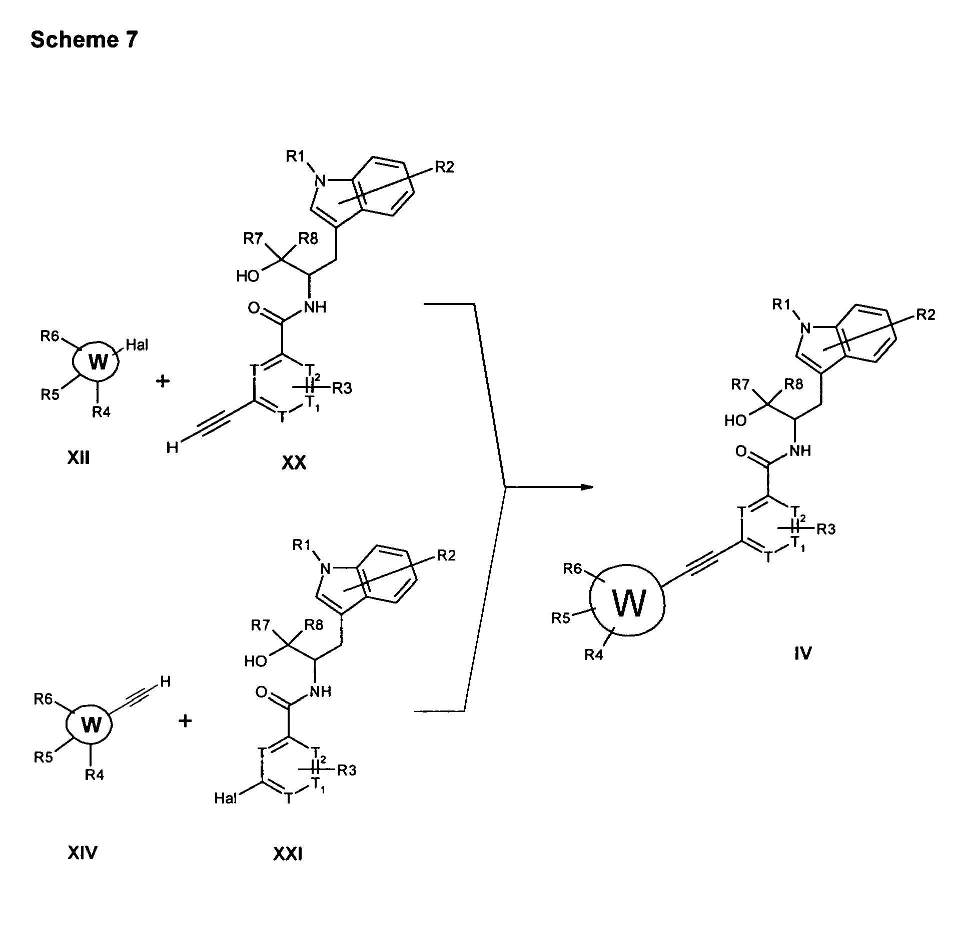

Likewise, compounds of the general formula V can be prepared as shown in Scheme 8 by a Sonogashira type coupling reaction between an acetylene derivative XXII or XIV and an aryl halide XII or XXII Reagents suitable for this purpose are palladium catalysts in combination with or without a Cu (I) source , with or without a base in a suitable solvent which are known to the skilled person.

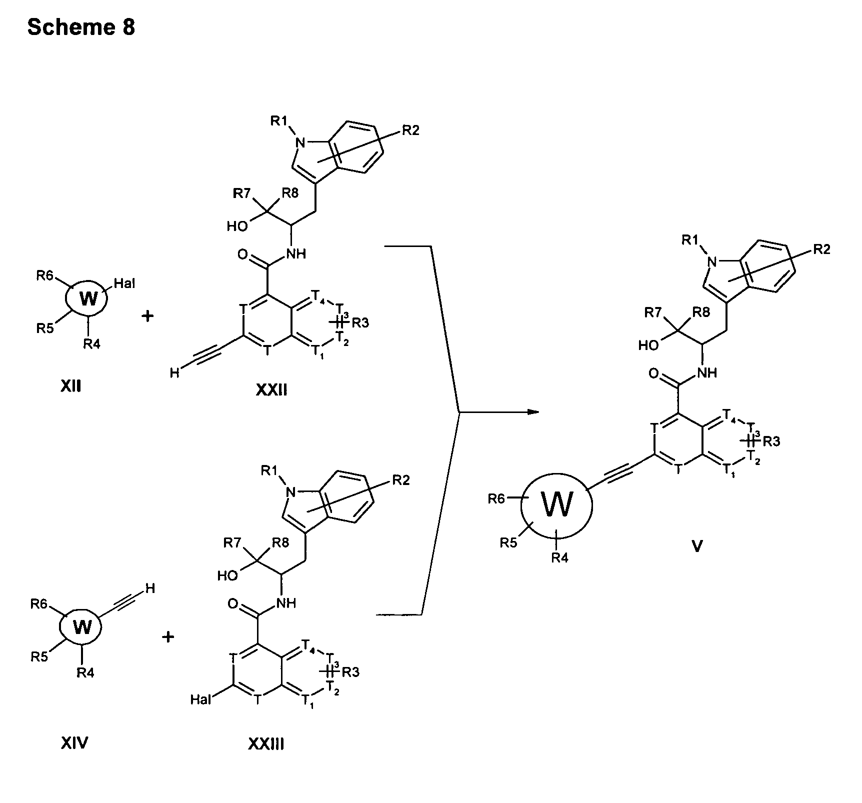

The present invention further relates to the carboxylic acids of the formulae VII, VIII, IX, X and XI as intermediates of the process according to the invention for preparing the compounds according to the invention, namely:
5-Phenylethynyl-2-propoxy-benzoic acid
5-(2-Methoxy-phenylethynyl)-2-propoxy-benzoic acid
2-Isopropoxy-5-(2-methoxy-phenylethynyl)-benzoic acid
and the methyl, ethyl, propyl and butyl esters thereof.

The present invention also relates to the aryl acetylenes of the formulae XIII, XVI, XVIII, XX and XXII as intermediates of the process according to the invention for preparing the compounds according to the invention, namely:
5-Ethynyl-N-[(R)-1-hydroxymethyl-2-(1H-indol-3-yl)-ethyl]-2-isopropoxy-benzamide
2-Ethoxy-5-ethynyl-N-[(R)-1-hydroxymethyl-2-(1H-indol-3-yl)-ethyl]-benzamide.

The present invention also relates to the aryl halides of the formulae XV, XVII, XIX, XXI and XXIII as intermediates of the process according to the invention for preparing the compounds according to the invention, namely:
3-Bromo-naphthalene-1-carboxylic acid [(R)-1-hydroxymethyl-2-(1H-indol-3-yl)-ethyl]-amide
6-Bromo-quinoline-8-carboxylic acid [(R)-1-hydroxymethyl-2-(1H-indol-3-yl)-ethyl]-amide

### Pharmacological data

### HTRF assay for measuring cAMP in cells

The method is based on a competitive immunoassay between native cAMP, which has been produced by the cells, and cAMP which is labelled with cAMPD2 conjugate. The tracer binding was visualized by a monoclonal antibody, anti-cAMP labelled with cryptate [HTRF = homogeneous time-resolved fluorescence].
The specific signal is inversely proportional to the cAMP concentration of the samples employed.
The 665nm/ 620nm fluorescence ratio was evaluated.

Materials used: 96-well plates for the tissue culture, 96-well plates with black edge and black base (e.g. Fluotrac 600 from Greiner), 96-well plates for the substance dilutions of polypropylene and cAMP Femtomolar (4000wells Kit, CIS Bio International # 62AM5PEJ).
Reagent used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.

Buffer 1 (washing and testing buffer) contained PBS, 1 mM CaCl2, 1 mM MgCl₂, 0.2% glucose; 0.1% BSA, 1 mM IBMX.

Buffer 2 (2x lysis buffer) contained 1% Triton X-100 in PBS (without CaCl₂ and MgCl₂).

Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells. The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min). The test conjugates (cAMP-D2 and anti-cAMP cryptate, CIS Bio) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of cAMP-D2 conjugate were pipetted and, after 10 min, 25 µl of the anti-cAMP cryptate were added). This is followed by incubation at RT for 90 minutes. The measurement was carried out in a PheraStar (BMG).

### Tissue culture conditions

| | |
|---|---|
| 1) hFSHr clone 16 | Ham's F12 PSG 10% FCS 700 µg/ml G 418 (Geneticin) from PAA. |

Dose-effect curve (hFSH) for the human receptor: 1 e-8, 3e-9, 1 e-9, 3e-10, 1e-10, 3e-11, 1 e-11, 3e-12 mol/I.
The test substances were employed in suitable dilutions in the absence (test for agonism) and in the presence of 1e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀. The test results (Table 1) show that the compounds according to the invention have an FSH-antagonistic effect.

**Table 1. FSH-antagonistic effect of selected compounds in the HTRF assay**

| Compound [Ex. #] | **IC₅₀** |
|---|---|
| 1 | 250 nM |
| 2 | 70 nM |
| 4 | 7.5 µM |
| 5 | 80 nM |
| 6 | 100 nM |
| 20 | 700 nM |
| 21 | 300 nM |
| 22 | 150 nM |
| 23 | 30 nM |
| 24 | 20 nM |
| 30 | 150 nM |
| 40 | 500 nM |
| 46 | 150 nM |
| 48 | 30 nM |
| 52 | 80 nM |
| 58 | 2,5 µM |

Being potent antagonists of the FSH receptor, compounds of the general formula I or pharmaceutically acceptable salts thereof can thus be used for the manufacture of medicaments to be used for the fertility control in male and/or in a female animals, in particular in men and/or women; as well as for the treatment and/or prevention of osteoporosis.

### Pharmaceutical compositions

The invention further relates to compounds of the general formula I or pharmaceutically acceptable salts thereof as therapeutic active ingredients, and to pharmaceutical compositions comprising at least one compound of the general formula I or pharmaceutically acceptable salts thereof, where appropriate together with pharmaceutically suitable excipients and/or carriers.

Pharmaceutically acceptable salts of the compounds of the general formula I can be prepared by methods known to the skilled person, depending on the nature of the compound of formula I, either by using as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid;
or by using an appropriate base as inorganic base inter alia alkalimetal hydroxide, carbonate, or hydrogencarbonate, as organic base inter alia tertiary amines and N-heterocycles.
These pharmaceutical compositions and medicaments may be intended for oral, rectal, subcutaneous, transdermal, percutaneous, intravenous or intramuscular administration. They comprise besides conventional carriers and/or diluents at least one compound of the general formula I.
The medicaments of the invention are formulated using the customary solid or liquid carriers or diluents and the excipients customarily used in pharmaceutical technology, in accordance with the desired mode of administration with a suitable dosage in a known manner: i.e. by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980) and to Gennaro, A. R. et al., Remington: The Science and Practice of Pharmacy (20th Edition., Lippincott Williams & Wilkins 2000, see especially Part 5: Pharmaceutical Manufactoring).
Pharmaceutical compositions according to the present invention are preferably administered orally. Suitable for oral administration are in particular tablets, (film)-coated tablets, sugar-coated tablets capsules, pills, powders, granules, pastilles, suspensions, emulsions, solutions or depot forms.
Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.
Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.
Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as *p*-hydroxybenzoates.
Capsules comprising the compounds of the general formula I can be produced for example by the compound(s) of the general formula I being mixed with an inert carrier such as lactose or sorbitol and encapsulated in gelatine capsules.

Parenteral preparations such as solutions for injection are also suitable. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy.
Suitable suppositories can be produced for example by mixing with carriers intended for this purpose, such as neutral fats or polyethylene glycol or derivatives thereof.
Formulations suitable for topical application include gels, ointments, greasy ointments, creams, pastes, dusting powders, milk and tinctures. Topical use can also take place by means of a transdermal system, for example a patch. The concentration of the compounds of the general formula I in these preparations should typically be in the range of 0.01% - 20% in order to achieve an adequate pharmacological effect.

The invention further relates to pharmaceutical compositions in combination with packaging material suitable for said composition, wherein said packaging material including instructions for the use of the composition.

### Dose

Suitable doses for the compounds according to the present invention may vary from 0.005 mg to 50 mg per day per kg of body weight, depending on the age and constitution of the patient. It is possible to administer the necessary daily dose by single or multiple delivery. The preferred daily dose for larger mammals, for example humans, may vary in the range from 10 µg to 30 mg per kg of body weight.
The exact dose and regimen of administration of the drug substance (active ingredient, or a pharmaceutical composition thereof, may however vary with the particular compound, the route of administration, and the age, sex and condition of the individual to whom the medicament is administered. The dose, the dosage as well as the regimen of the administration may thus differ between a male and a female considerably.

The compounds according to the invention of the general formula I can be prepared as described below.

### Abbreviations used:

- ACN: Acetonitrile
- DIBAH: Diisobutylaluminium hydride
- DMF: *N*,*N*-Dimethylformamide
- EDC: *N*-Ethyl-*N*'-(3-dimethylaminopropyl)carbodiimide
- EtOH: Ethanol
- HATU: O-(7-Azabenzotriazol-1-yl)-N,N,N',N'-tetramethyluronium hexafluorophosphate
- FMOC: (9*H*-Fluoren-9-ylmethoxy)carbonyl
- HOBt: 1-Hydroxy-1*H*-benzotriazole
- MeCN: Acetonitrile
- MeOH: Methanol
- MTBE: Methyl tert-butyl ether
- NMM: 4-methylmorpholine
- NMP: N-Methylpyrrolidinone
- Rf: Reflux
- RT: Room temperature
- TBAF: Tetrabutylammonium fluoride
- TFA: Trifluoroacetic acid
- THF: Tetrahydrofuran

Compounds of the general formula I can in principle be prepared as shown in Scheme 9 by an amide forming reaction between a tryptophanol derivative VI and a carboxylic acid VII. The reagents typically used for the coupling are EDC and HOBt. 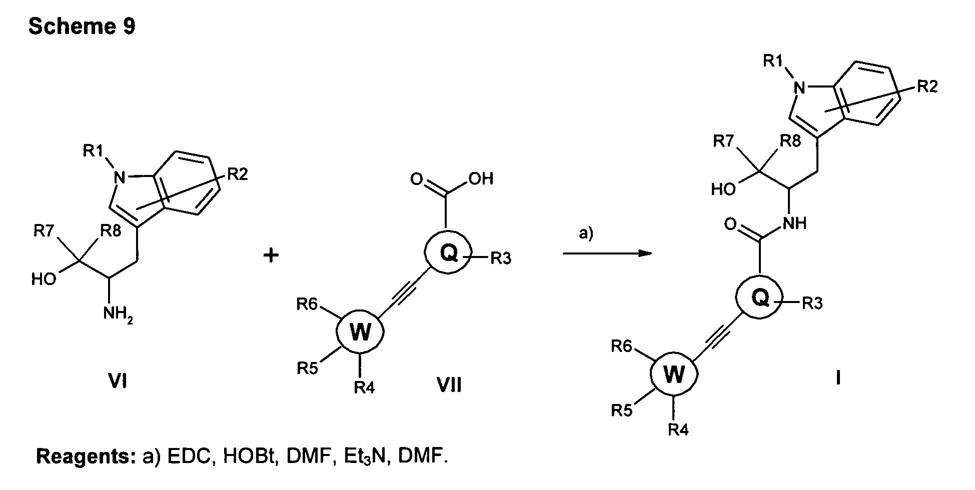 The tryptophanol derivatives of the formula VI can in principle be prepared as shown in Scheme 10 from the corresponding amino acids which can be purchased or are known from the literature. 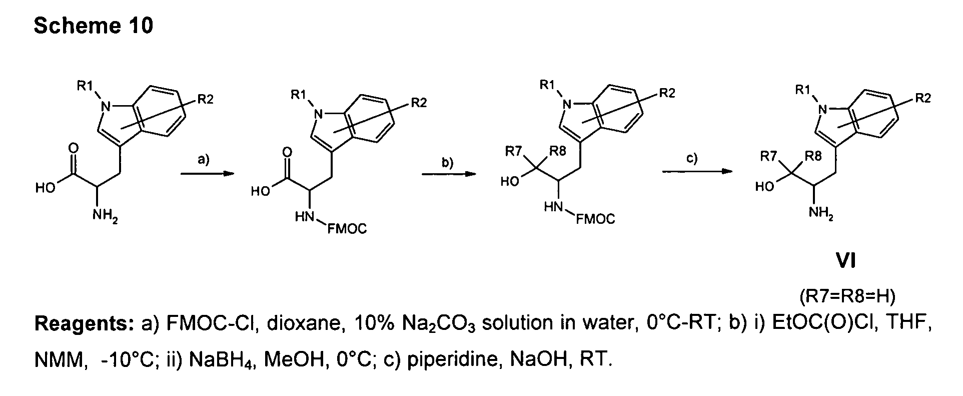 The tryptophanol derivatives of the general formula I can in principle be also prepared according to Scheme 11 from the corresponding amino acids esters via Grignard addition or reduction with lithium borohydride.

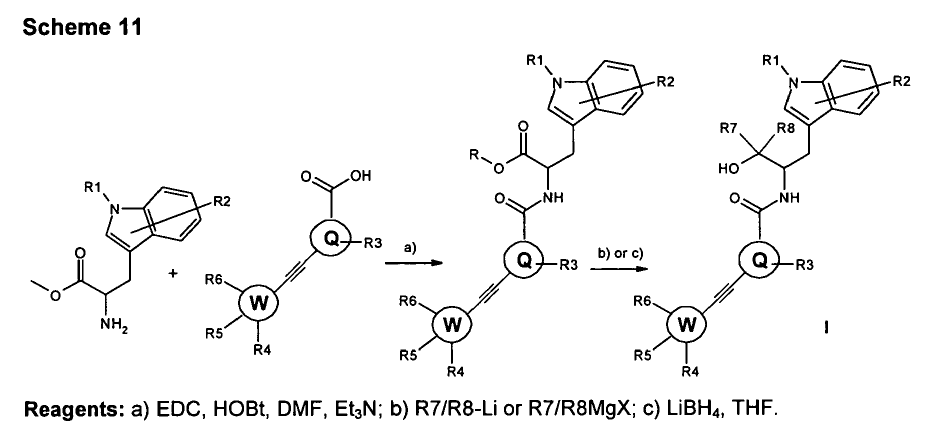

The carboxylic acid derivatives of formula VII can in principle be prepared according to Scheme 12 via a Sonogashira type coupling of acetylenes with their corresponding aryl halides with subsequent hydrolysis of the resulting carboxylic esters.

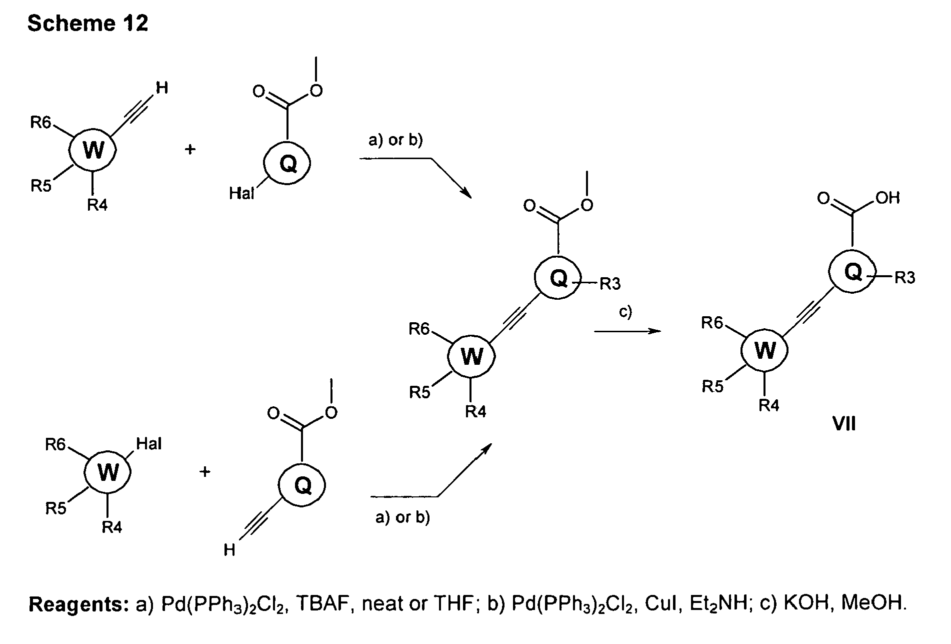

The acetylene derivatives of formula I can in principle also be prepared according to Scheme 13 via a Sonogashira type coupling of terminal acetylenes with their corresponding aryl halides.

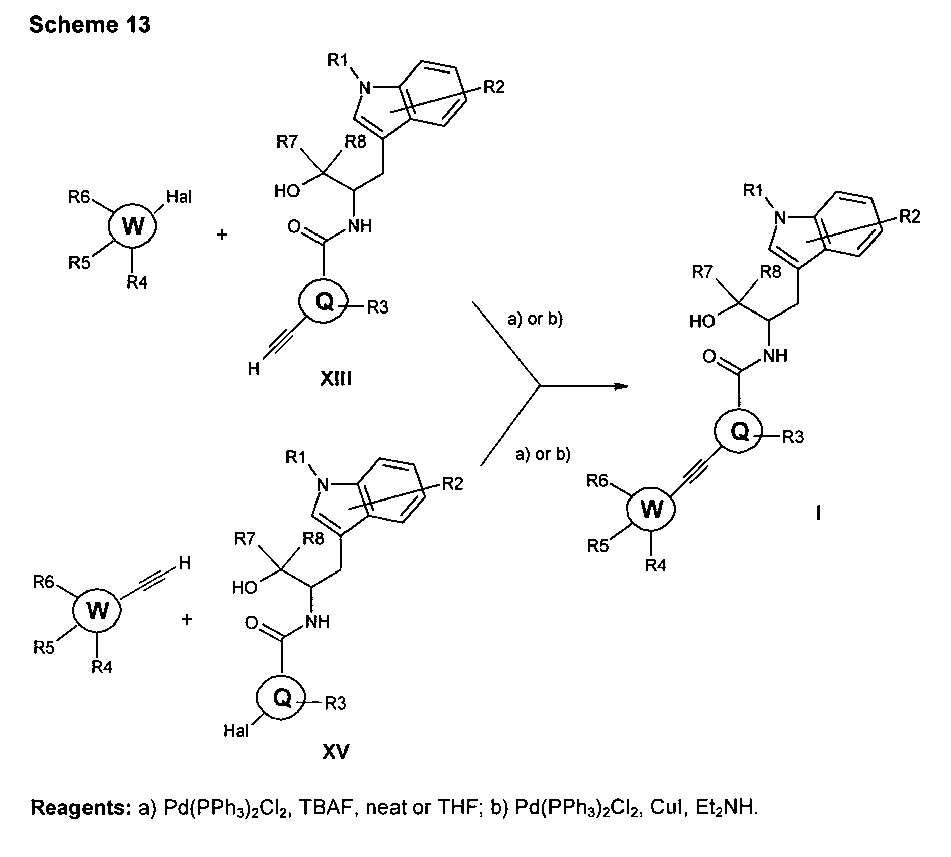

The acetylene derivatives of formula XIII can in principle be prepared according to Scheme 14 via a Sonogashira type coupling of trimethylsilyl acetylene with aryl halide XV followed by deprotection of the silyl group using TBAF .

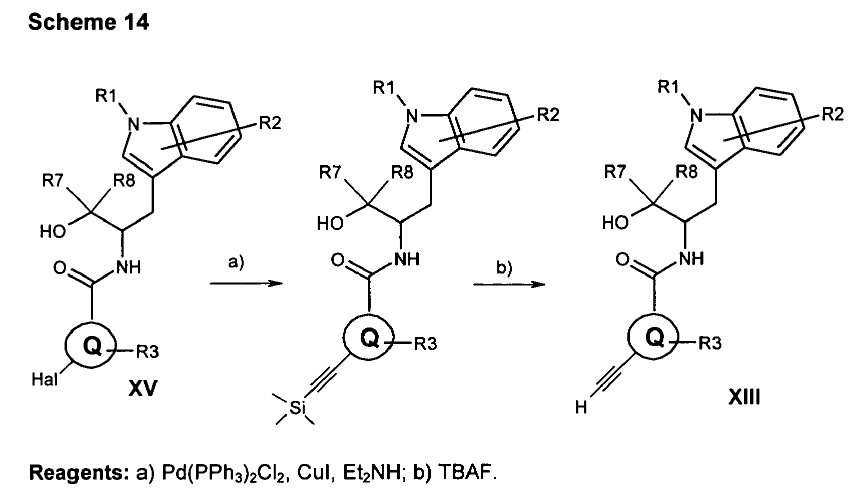

Compounds of the general formula XV can in principle be prepared according to Scheme 15 by an amide forming reaction between a tryptophanol derivative Vl and their corresponding carboxylic acid. The reagents typically used for the coupling are EDC and HOBt.

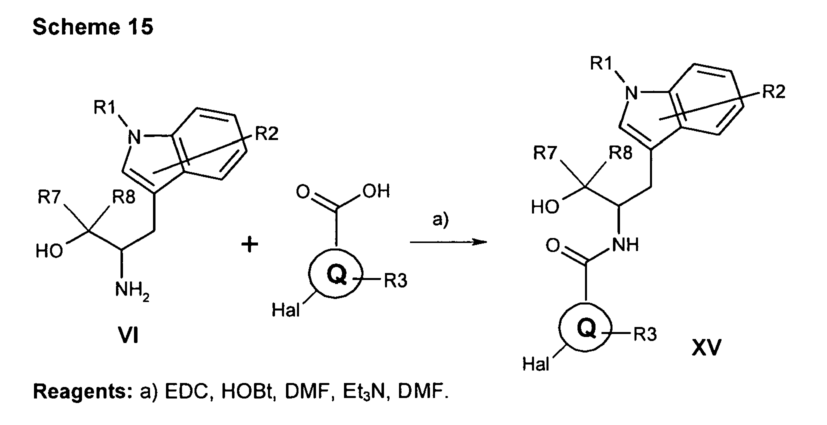

### Synthesis of the compounds according to the invention

### Example 1

### N-[(R)-1-Hydroxymethyl-2-(1H-indol-3-yl)-ethyl]-5-phenylethynyl-2-propoxybenzamide

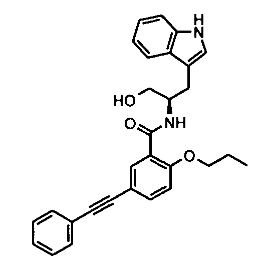

### 1a) 5-lodo-2-propoxy-benzoic acid methyl ester

A suspension of 5-lodo-2-hydroxy benzoic acid methyl ester (100 g), potassium carbonate (149 g) and n-propyliodide (180 ml) in acetone (1000 ml) was stirred under reflux overnight. The reaction mixture was allowed to cool down to ambient temperature and the solid was removed by filtration. The filtrate was evaporated and the title compound was crystallized at -50°C from petrol ether. Yield 68 % (78 g). **¹H-NMR (CDCl₃):** 8.05 d (*J* = 2.5 Hz, 1H); 7.67 dd (*J* = 2.3 Hz / 8.7 Hz, 1H); 6.72 d (*J* = 8.9 Hz, 1H); 3.96 m (2H); 3.87 s (3H); 1.83 m (2H); 1.05 m (3H).

### 1b) 5-Phenylethynyl-2-propoxy-benzoic acid methyl ester

5-lodo-2-propoxy-benzoic acid methyl ester (500 mg), phenyl acetylene (0.34 ml), palladium dichlorobis(triphenylphosphine) (11 mg) and Cul (5 mg) in diethylamine (10 ml) were stirred at 60°C for 12 hours. The reaction mixture was concentrated and extracted with ethylacetate / water. The combined organic layers were dried over sodium sulphate and the solvent was evaporated. The title compound was obtained in 62 % yield (285 mg) after flash chromatography. **¹H-NMR (DMSO-d₆):** 7.76 d (*J* = 2.3 Hz, 1H); 7.64 dd (*J* = 2.3 Hz /8.6 Hz, 1H); 7.50 m (2H); 7.37 m (3H); 7.16 d (*J* = 8.9 Hz, 1H); 4.01 m (2H); 3.77 s (3H); 1.70 m (2H); 0.96 m (3H).

### 1c) 5-Phenylethynyl-2-propoxy-benzoic acid

A solution of 5-Phenylethynyl-2-propoxy-benzoic acid methyl ester (280 mg) in methanolic KOH solution (10%) was stirred at ambient temperature overnight.The solvent was distilled off and the remaining solid was dissolved in water and extracted with ethylacetate. The water phase was acidified by addition of HCl (2 molar) and the water phase was extracted with ethylacetate (3x 50 ml). The combined organic layers were dried over sodium sulphate and evaporated to dryness. The title compound was obtained in 85% yield (227 mg). **¹H-NMR (DMSO-d₆):** 12,75 s (1H); 7,72 d (*J* = 2,3 Hz, 1H); 7,61 dd (*J* = 2,3 Hz / 8,6 Hz, 1H); 7,50 m (2H); 7,37 m (3H); 7,12 d (*J* = 8,9 Hz, 1H); 4,00 m (2H); 1,70 m (2H); 0,96 m (3H).

### 1d) N-[(R)-1-Hydroxymethyl-2-(1H-indol-3-yl)-ethyl]-5-phenylethynyl-2-propoxybenzamide

A solution of 5-phenylethynyl-2-propoxy-benzoic acid (200 mg), (R)-tryptophanol (163 mg), HOBt monohydrate (131 mg) and EDC (164 mg) in DMF (10 ml) was stirred overnight at ambient temperature. The reaction mixture was poured into water and the resulting precipitate was filtered off. After drying the title compound was obtained in 76 % yield (245 mg). **¹H-NMR (DMSO-d₆):** 10,82 s (1H); 8,27 d (*J* = 8,1Hz, 1H); 8,01 d (*J* = 2,3 Hz, 1H); 7,67 m (2H); 7,55 m (2H); 7,43 m (3H); 7,34 d (*J* = 7,9 Hz, 1H); 7,20 d (*J* = 8,9 Hz, 1H); 7,17 m (1H); 7,07 m (1H); 6,97 m (1H); 4,92 m (1H); 4,27 m (1H); 4,05 m (2H); 3,50 m (2H); 2,99 m (2H); 1,68 m (2H); 0,94 m (3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **2** | N-[(R)-1-Hydroxymethyl-2-(1H-indol-3-yl)-ethyl]-5-(2-methoxyphenylethynyl)-2-propoxy-benzamide; | 1 | **¹H-NMR (DMSO-d₆):** 10.79 s(1H); 8.24 d (*J* = 8.3 Hz, 1H); 7.93 d (*J* = 2.3 Hz, 1H); 7.63 d (*J* = 7.7 Hz, 1H); 7.56 dd (*J* = 2.3 Hz / 8.5 Hz, 1H); 7.43 dd (*J* = 1.7 Hz / 7.5 Hz, 1H); 7.33 m (2H); 7.14 m (2H); 7.05 m (2H); 6.93 m (2H); 4.89 m (1H); 4.22 m (1H); 4.00 m (2H); 3.83 s (3H); 3.44 m (2H); 2.93 m (2H); 1.63 m (2H); 0.89 m (3H). | 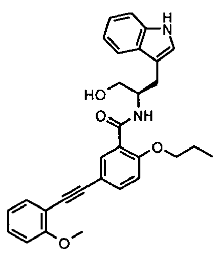 |
| | | | | |
| | *(D)-Tryptophanol* | | | |
| | | | | |
| | *and* | | | |
| | | | | |
| | *5-(2-Methoxy-phenylethynyl)-2-propoxy-benzoic acid* | | | |
| **3** | N-[(R)-1-Hydroxymethyl-2-(1H-indol-3-yl)-ethyl]-2-isopropoxy-5-(2-methoxy-phenylethynyl)-benzamide; | **1** | **¹H-NMR (CDCl₃):** 8.47 d (*J* = 7.4 Hz, 1H); 8.44 d (*J* = 2.3 Hz, 1H); 8.23 s (1H); 7.70 d (*J* = 7.5 Hz, 1H); 7.57 dd (*J* = 2.3 Hz / 8.5 Hz, 1H); 7.48 dd (*J* = 1.7 Hz / 7.5 Hz, 1H); 7.33 m (2H); 7.10 m (3H); 6.91 m (3H); 4.64 m (1H); 4.56 m (1H); 4.12 m (1H); 3.91 s (3H); 3.80 m (2H); 3.13 m (2H); 1.25 d (*J* = 6.0 Hz, 3H); 1.19 d (*J* = 6.0 Hz, 3H). | 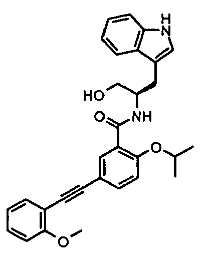 |
| | | | | |
| | *(D)-Tryptophanol* | | | |
| | | | | |
| | *and* | | | |
| | | | | |
| | *2-Isopropoxy-5-(2-methoxyphenylethynyl)-benzoic acid* | | | |
| **4** | N-[(S)-1-Hydroxymethyl-2-(1H-indol-3-yl)-ethyl]-2-isopropoxy-5-(2-methoxy-phenylethynyl)-benzamide; | **1** | **¹H-NMR (CDCl₃):** 8.47 d (*J* = 7.4 Hz, 1H); 8.44 d (*J* = 2.3 Hz, 1H); 8.23 s (1H); 7.70 d (*J* = 7.5 Hz, 1H); 7.57 dd (*J* = 2.3 Hz / 8.5 Hz, 1H); 7.48 dd (*J* = 1.7 Hz / 7.5 Hz, 1H); 7.33 m (2H); 7.10 m (3H); 6.91 m (3H); 4.64 m (1H); 4.56 m(1H); 4.12 m (1H); 3.91 s (3H); 3.80 m (2H); 3.13 m (2H); 1.25 d (*J* = 6.0 Hz, 3H); 1.19 d (*J* = 6.0 Hz, 3H). | 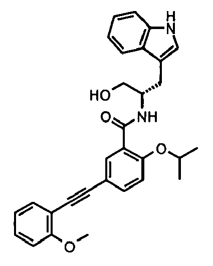 |
| | | | | |
| | *(L)-Tryptophanol* | | | |
| | | | | |
| | *and* | | | |
| | | | | |
| | *2-Isopropoxy-5-(2-methoxy-phenylethynyl)-benzoic acid* | | | |

### Example 5

### N-[(R)-1-Hydroxymethyl-2-(1H-indol-3-yl)-ethyl]-2-isopropoxy-5-o-tolylethynyl-benzamide

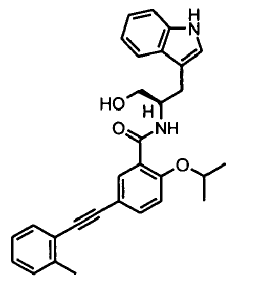

### 5a) 5-Iodo-2-isopropoxy-benzoic acid methyl ester

A suspension of 5-Iodo-2-hydroxy benzoic acid methyl ester (50 g), potassium carbonate (74,6 g) and *iso*-propyl iodide (89,9 ml) in acetone (500 ml) was stirred under reflux overnight. The reaction mixture was allowed to cool down to ambient temperature and the solid was removed by filtration. The filtrate was evaporated and the title compound was obtained in 96 % yield (55,1 g). **¹H-NMR (CDCl₃):** 8.02 d (*J* = 2.3 Hz, 1H); 7.67 dd (*J* = 2.5 Hz / 8.9 Hz, 1H); 6.74 d (*J* = 8.9 Hz, 1H); 4.54 m (1H); 3.87 s (3H); 1.36 m (6H).

### 5b) 2-Isopropoxy-5-trimethylsilanylethynyl-benzoic acid methyl ester

5-Iodo-2-isopropoxy-benzoic acid methyl ester (25 g), palladium di-chlorobis(triphenylphosphine) (2.74) and Cul (14.87 g) in diethylamine (250 ml) were stirred at ambient temperature overnight. The reaction mixture was concentrated and extracted with ethylacetate / water. The combined organic layers were dried over sodium sulphate and the solvent was evaporated. The title compound was obtained in 81 % yield (18.4 g) after flash chromatography. **¹H-NMR (CDCl₃):** 7.88 d (*J* = 2.3 Hz, 1H); 7.50 dd (*J* = 2.3 Hz / 8.7 Hz, 1H); 6.89 d (*J* = 8.7 Hz, 1H); 4.60 m (1H); 3.87 s (3H); 1.37 m (6H); 0.23 s (9H).

### 5c) 5-Ethynyl-2-isopropoxy-benzoic acid

A solution of 2-isopropoxy-5-trimethylsilanylethynyl-benzoic acid methyl ester (7.79 g) in was stirred in methanolic KOH solution (100 ml, 10%) under reflux overnight. The solvent was distilled off and the remaining solid was dissolved in water and extracted with ethylacetate. The water phase was acidified by addition of HCl (2 molar) and the water phase was extracted with ethylacetate (3x 200 ml). The combined organic layers were dried over sodium sulphate and evaporated to dryness. The title compound was obtained in 97 % yield (5.29 g). **¹H-NMR (DMSO-d₆):** 12.70 s (1H); 7.60 d (*J* = 2.3 Hz, 1H); 7.51 dd (*J* = 2.3/8.6 Hz, 1H); 7.10d (*J* = 8.6 Hz, 1H); 4.65 m (1H); 4.05 s (1H); 1.23 m (6H).

### 5d) 5-Ethynyl-N-[(R)-1-hydroxymethyl-2-(1H-indol-3-yl)-ethyl]-2-isopropoxy-benzamide

A solution of 5-Ethynyl-2-isopropoxy-benzoic acid (240 mg), (R)-tryptophanol (268 mg), HOBt (191 mg) and EDC (270 mg) in DMF (10 ml) was stirred overnight at ambient temperature. The reaction mixture was concentrated and extracted with ethylacetate / water. The combined organic layers were dried over sodium sulphate and the solvent was evaporated. The title compound was obtained in 54 % yield (240 mg) after flash chromatography. **¹H-NMR (DMSO-d₆):** 10.78 s (1H); 8.32 d (*J* = 8.1 Hz, 1H); 7.90 d (*J* = 2.3 Hz, 1H); 7.60 d (*J* = 7.7 Hz, 1H); 7.51 dd (*J* = 2.5 Hz / 8.7 Hz, 1H); 7.28 d (*J* = 7.9 Hz, 1H); 7.15 d (*J* = 8.9 Hz, 1H); 7.11 d (*J* = 2.3 Hz, 1H); 7.02 m (1H); 6.93 m (1H); 4.90 m (1H); 4.75 m (1H); 4.18 m (1H); 4.06 s (1H); 3.42 m (2H); 2.92 m (2H); 1.20 m (6H).

### 5e) N-[(R)-1-Hydroxymethyl-2-(1H-indol-3-yl)-ethyl]-2-isopropoxy-5-o-tolylethynyl-benzamide

2-lodo-toluene (68 µl), 5-Ethynyl-N-[(R)-1-hydroxymethyl-2-(1H-indol-3-yl)-ethyl]-2-isopropoxy-benzamide (100 mg), palladium dichlorobis(triphenylphosphine) (9.3 mg) and Cul (10 mg) in diethylamine (10 ml) were stirred under reflux for 16 hours. The reaction mixture was concentrated and extracted with ethylacetate / water. The combined organic layers were dried over sodium sulphate and the solvent was evaporated. The title compound was obtained in 49 % yield (61 mg) after flash chromatography. **¹H-NMR (CDCl₃):** 8.52 d (*J* = 7.5 Hz, 1H); 8.43 d (*J* = 2.3 Hz, 1H); 8.40 s (1H); 7.73 d (*J* = 8.3 Hz, 1H); 7.54 dd (*J* = 2.5 Hz / 8.7 Hz, 1H); 7.49 d (*J* = 6.4 Hz, 1H); 7.34 d (*J* = 7.7 Hz, 1H); 7.24-7.09 m (5H); 7.05 d (*J* = 2.3 Hz, 1H); 6.89 d (*J* = 8.9 Hz, 1H); 4.64 m (1H); 4.60 m (1H); 3.77 m (2H); 3.14 m (2H); 2.51 s (3H); 1.26 m (3H); 1.20 m (3H).

### Example 6

### 3-(2-Methoxy-phenylethynyl)-naphthalene-1-carboxylic acid [(R)-1-hydroxymethyl-2-(1H-indol-3-yl)-ethyl]-amide

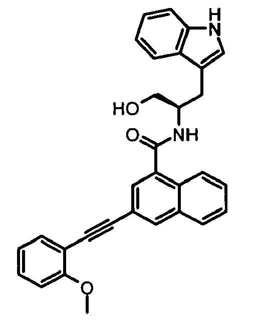

### 6a) 3-Bromo-naphthalene-1-carboxylic acid [(R)-1-hydroxymethyl-2-(1H-indol-3-yl)-ethyl]-amide

3-Bromo-naphthalene-1-carboxylic acid (500 mg) were stirred under reflux in SOCl₂ (10 ml) for 5 hours. The SOCl₂ was distilled off under vacuum and the residue was dissolved in CH₂Cl₂ (10 ml). This solution was added at 0°C to a solution of (D)-tryptophanol (378 mg) and triethylamin (0.55 ml) in dichlormethane (10 ml). The ice bath was removed and the reaction mixture was allowed to stir at ambient temperature overnight. The solvent was removed under vacuum and the residue was extracted with water / ethylacetate (3 x 50 ml). The combined organic layers were dried over sodium sulphate and the solvent was removed under vacuum. The title compound could be crystallized from diisopropylether (yield: 77 %, 650 mg). **¹H-NMR (DMSO-d₆):** 10.80 s (1H); 8.41 d (*J* = 8.5 Hz, 1H); 8.22 d (*J* = 1.9 Hz, 1H); 7.88 m (2H); 7.61 d (*J* = 7.7 Hz, 1H); 7.51 m (2H); 7.48 m (1H); 7.32 d (*J* = 8.1 Hz, 1H); 7.14 s (1H); 7.04 m (1H); 6.95 m (1H); 4.82 m (1H); 4.32 m (1H); 3.52 m (2H); 3.00 m (1H); 2.87 m (1H).

### 6b) 3-(2-Methoxy-phenylethynyl)-naphthalene-1-carboxylic acid [(R)-1-hydroxymethyl-2-(1H-indol-3-yl)-ethyl]-amide

3-Bromo-naphthalene-1-carboxylic acid [(R)-1-hydroxymethyl-2-(1H-indol-3-yl)-ethyl]-amide (200 mg), 1-Ethynyl-2-methoxy-benzene (122 µl), palladium dichlorobis(triphenylphosphine) (16,6 mg) and Cul (10 mg) in diethylamine (10 ml) were stirred under reflux overnight. The reaction mixture was concentrated and extracted with ethylacetate / water. The combined organic layers were dried over sodium sulphate and the solvent was evaporated. The title compound was obtained in 12 % yield (27 mg) after HPLC purification. **¹H-NMR (DMSO-d₆):** 10.81 s (1H); 8.43 d (*J* = 8.6 Hz, 1H); 8.15 s (1H); 7.94 d (*J* = 7.6 Hz, 1H); 7.89 d (*J* = 8.3 Hz, 1H); 7.64 d (*J* = 7.8 Hz, 1H); 7.51 m (4H); 7.38 m (1H); 7.31 d (*J* = 8.1 Hz, 1H); 7.15 s (1H); 7.11 d (*J* = 8.3 Hz, 1H); 6.96 m (3H); 4.84 s (1H); 4.33 m (1H); 3.85 s (3H); 3.55 m (2H); 3.00 m (1H); 2.87 m (1H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **7** | 6-(2-Methoxy-phenylethynyl)-quinoline-8-carboxylic acid [(R)-1-hydroxymethyl-2-(1H-indol-3-yl)-ethyl]-amide; | **6** | **¹H-NMR (DMSO-d₆):** 10.97 d (*J* = 7.91Hz, 1H); 10.79 s (1H); 8.88 dd (*J* = 1.9 Hz / 4.3 Hz, 1H); 8.53 m (2H); 8.35 d (*J* = 2.1 Hz, 1H); 7.67 m (2H); 7.54 dd (*J* = 1.7 Hz/7.7 Hz, 1H); 7.41 m (1H); 7.29 d (*J* = 8.1 Hz, 1H); 7.22 d (*J* = 2.3 Hz, 1H); 7.10 d (*J* = 8.1 Hz, 1H); 7.04-6.89 m (3H); 4.94 m (1H); 4.36 m (1H); 3.87 s (3H); 3.57 m (1H); 3.50 m (1H); 3.08 m (2H). | 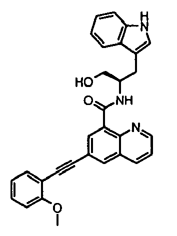 |
| | | | | |
| | *6-Bromo-quinoline-8-carboxylic acid [(R)-1-hydroxymethyl-2-(1H-indol-3-yl)-ethyl]-amide* | | | |
| | | | | |
| | *and* | | | |
| | | | | |
| | *1-Ethynyl-2-methoxy-benzene* | | | |

### Example 8

### N-[(R)-1-Hydroxymethyl-2-(1H-indol-3-yl)-ethyl]-2-isopropoxy-5-(3-ethylcarbamoylphenylethynyl)-benzamide

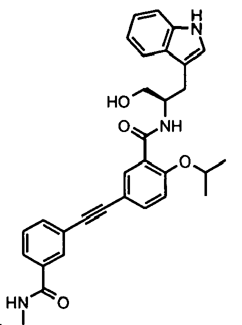

3-lodo-N-methyl-benzamide (58 mg), 5-Ethynyl-N-[(R)-1-hydroxymethyl-2-(1H-indol-3-yl)-ethyl]-2-isopropoxy-benzamide (100 mg), palladium dichlorobis(triphenylphosphine) (4.7 mg) and TBAF x 3 H₂O (170 mg in THF (5 ml) were stirred at 80°C for 5 hours. The reaction mixture was concentrated and extracted with ethylacetate / water. The combined organic layers were dried over sodium sulphate and the solvent was evaporated. The title compound was obtained in 79 % yield (89 mg) after flash chromatography. **¹H-NMR (CDCl₃):** 8.48 (*J* = 7.3 Hz, 1H); 8.39 d (*J* = 2.0 Hz, 1H); 8.20 s (1H); 7.87 s (1H); 7.77 d (*J* = 6.6 Hz, 1H); 7.72 d (*J* = 7.6 Hz, 1H); 7.61 d (*J* = 6.6 Hz, 1H); 7.52 dd (J = 2.3 Hz / 8.6 Hz, 1H); 7.4 m (2 H); 7.19 m (1H); 7.09 m (2H); 6.90 d (*J* = 8.6 Hz, 1H); 4.66 m (1H); 4.57 m (1H); 3.81 m (2H); 3.14 m (2H); 3.03 d (*J* = 4.3 Hz, 3H); 1.27 m (3H); 1.21 m (3H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **9** | 2-Ethoxy-N-[(R)-1-hydroxy-methyl-2-(1H-indol-3-yl)-ethyl]-5-(3-methylcarbamoyl-phenylethynyl)-benzamide; | **8** | **¹H-NMR (DMSO-d₆):** 10.79 s (1H); 8.52 d (*J* = 4.6 Hz, 1H); 8.28 m (2H); 7.98 m (2H); 7.81 d (*J* = 7.8 Hz, 1H); 7.64 m (3H); 7.48 m(1H); 7.30 d (*J* = 8.1 Hz, 1H); 7.15 d (*J* = 8.6 Hz, 1H); 7.12 d (*J* = 2.3 Hz, 1H); 7.02 m (1H); 6.93 m(1H); 4.89 m (1H); 4.21 m (1H); 4.12 m (2H); 3.43 m (2H); 2.94 m (2H); 2.75 d (*J* = 4.6 Hz, 3H); 1.26 m (3H). | 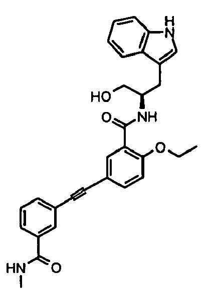 |
| | | | | |
| | *2-Ethoxy-5-ethynyl-N-[(R)-1-hydroxymethyl-2-(1H-indol-3-yl)-ethyl]-benzamide* | | | |
| | | | | |
| | *and* | | | |
| | | | | |
| | *3-Iodo-N-methyl-benzamide* | | | |
| **10** | N-[(R)-1-Hydroxymethyl-2-(1H-indol-3-yl)-ethyl]-2-isopropoxy-5-(4-methylcarbamoyl-phenylethynyl)-benzamide; | **8** | **¹H-NMR (DMSO-d₆):** 10.78 s (1H); 8.49 d (*J* = 4,7 Hz, 1H); 8.34 d (*J* = 8.1 Hz, 1H); 8.01 d (*J* = 2.5 Hz, 1H); 7.82 d (*J* = 8.7 Hz, 2H); 7.61 m (3H); 7.28 d (*J* = 8.1 Hz, 1H); 7.22 d (*J* = 8.9 Hz, 1H); 7.12 d (*J* = 1.9 Hz, 1H); 7.02 m (1H); 6.93 m (1H); 4.91 m (1H); 4.79 m (1H); 4.20 m(1H); 3.44 m (2H); 2.94 m (2H); 2.75 d (*J* = 4.7 Hz, 3H); 1.22 m (6H). | 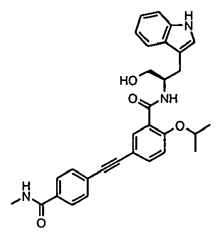 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *4-lodo-N-methyl-benzamide* | | | |
| **11** | N-[(R)-1-Hydroxymethyl-2-(1H-indol-3-yl)-ethyl]-2-isopropoxy-5-(1-oxo-1,2,3,4-tetrahydro-isoquinolin-7-ylethynyl)-benzamide; | **8** | **¹H-NMR (DMSO-d₆):** 10.79 s (1H); 8.45 d (*J* = 8.1 Hz, 1H); 8.28 s (1H); 8.19 d (*J* = 2.6 Hz, 1H); 8.05 d (*J* = 2.1 Hz, 1H); 7.98 s (1H); 7.74 m (2H); 7.72 d (*J* = 8.3 Hz, 1H); 7.36 d (*J* = 7.9 Hz, 1H); 7.29 d (*J* = 7.9 Hz, 1H); 7.24 d (*J* = 9.1 Hz, 1H); 7.12 d (*J* = 2.1 Hz, 1H); 7.03 m (1H); 6.94 m (1H); 4.92 m (1H); 4.78 m (1H); 4.22 m (1H); 3.45 m (2H); 3.37 m (2H); 2.91 m (4H); 1.24 m (6H). | 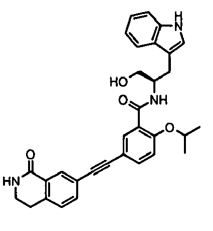 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *7-Bromo-3, 4-dihydro-2H-isoquinolin-1-one* | | | |
| **12** | 2-Ethoxy-N-[(R)-1-hydroxymethyl-2-(1H-indol-3-yl)-ethyl]-5-(4-methylcarbamoyl-phenylethynyl)-benzamide; | **8** | **¹H-NMR (DMSO-d₆):** 10.83 s (1H); 8.55 d (*J* = 4.5 Hz, 1H); 8.33 d (*J* = 8.3 Hz, 1H); 8.02 d (*J* = 2.3 Hz, 1H); 7.89 d (*J* = 8.5 Hz, 2H); 7.66 m (4H); 7.35 d (*J* = 8.1 Hz, 1H); 7.20 d (*J* = 8.7 Hz, 1H); 7.17 d (*J* = 1.9 Hz, 1H); 7.07 m (1H); 6.98 m (1H); 4.93 m (1H); 4.25 m (1H); 4.16 m (2H); 3.47 m (2H); 2.98 m (2H); 2.81 d (*J* = 4.5 Hz, 3H); 1.31 m (3H). | 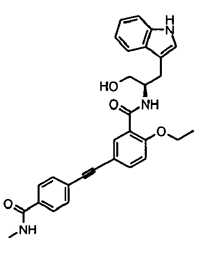 |
| | | | | |
| | *2-Ethoxy-5-ethynyl-N-[(R)-1-hydroxymethyl-2-(1H-indol-3-yl)-ethyl]-benzamide* | | | |
| | | | | |
| | *and* | | | |
| | | | | |
| | *4-lodo-N-methyl-benzamide* | | | |
| **13** | 5-(2,3-Dihydro-1H-indol-5-ylethynyl)-N-[(R)-1-hydroxymethyl-2-(1H-indol-3-yl)-ethyl]-2-isopropoxy-benzamide; | **8** | **¹H-NMR (DMSO-d₆):** 10.78 s (1H); 8.35 d (*J* = 8.1 Hz, 1H); 7.90 s (1H); 7.64 d (*J* = 7.8 Hz, 1H); 7.47 dd (*J* = 8.6 Hz / 2.3 Hz, 1H); 7.30 d (*J* = 8.1 Hz, 1H); 7.12 m (3H); 7.02 m (2H); 6.93 m (1H); 6.43 d (*J* = 8.1 Hz, 1H); 4.91 m (1H); 4.73 m (1H); 4.20 m (1H); 3.43 m (4H); 2.89 m (4H); 1.22 m (6H). | 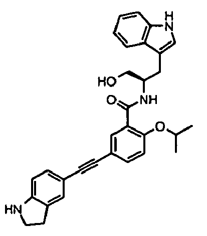 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *5-Bromo-2,3-dihydro- 1H-indole* | | | |
| **14** | 5-(5-Cyanomethyl-2-methoxy-phenylethynyl)-N-[(R)-1-hydroxymethyl-2-(1H-indol-3-yl)-ethyl]-2-isopropoxy-benzamide; | **8** | **¹H-NMR (DMSO-d₆):** 10.78 s (1H); 8.35 d (*J* = 8.1 Hz, 1H); 7.96 d (*J* = 2.5 Hz, 1H); 7.64 d (*J* = 6.8 Hz, 1H); 7.59 m (1H); 7.54 d (*J* = 2.5 Hz, 1H); 7.43 d (*J* = 2.5 Hz, 1H); 7.30 d (*J* = 8.1 Hz, 1H); 7.19 d (*J* = 9.0 Hz, 1H); 7.11 d (*J* = 2.1 Hz, 1H); 7.09 d (*J* = 8.5 Hz, 1H); 7.02 m (1H); 6.93 m (1H); 4.91 m (1H); 4.78 m (1H); 4.20 m (1H); 3.93 s (2H); 3.83 s (3H); 3.45 m (2H); 2.94 m (2H); 1.22 m (6H). | 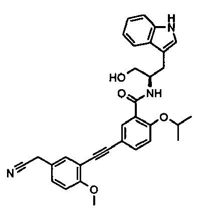 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *(3-Bromo-4-methoxy-phenyl)-acetonitrile* | | | |
| **15** | 5-(2,3-Dimethyl-phenylethynyl)-N-[(R)-1-hydroxymethyl-2-(1H-indol-3-yl)-ethyl]-2-isopropoxy-benzamide; | **8** | **¹H-NMR (DMSO-d₆):** 10.78 s (1H); 8.36 d (*J* = 8.3 Hz, 1H); 7.99 d (*J* = 2.3 Hz, 1H); 7.65 d (*J* = 7.7 Hz, 1H); 7.60 dd (*J* = 8.5 Hz / 2.3 Hz, 1H); 7.31 m (2H); 7.15 m (4H); 7.02 m (1H); 6.93 m (1H); 4.91 m (1H); 4.78 m (1H); 4.20 m (1H); 3.45 m (2H); 2.94 m (2H); 2.39 s (3H); 2.24 s (3H); 1.22 m (6H). | 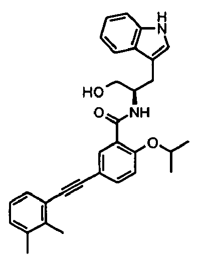 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-2,3-dimethyl-benzene* | | | |
| **16** | 5-(2-Cyano-thiophen-3-ylethynyl)-N-[(R)-1-hydroxymethyl-2-(1H-indol-3-yl)-ethyl]-2-isopropoxy-benzamide; | **8** | **¹H-NMR (DMSO-d₆):** 10.82 s (1H); 8.14 d (*J* = 5.1 Hz, 1H); 8.07 d (*J* = 2.5 Hz, 1H); 7.68 m (2H); 7.48 d (*J* = 5.3 Hz, 1H); 7.34 d (*J* = 8.1 Hz, 1H); 7.28 d (*J* = 8.9 Hz, 1H); 7.16 d (*J* = 2.1 Hz, 1H); 7.07 m (1H); 6.98 m (1H); 4.95 m (1H); 4.85 m (1H); 4.26 m (1H); 3.49 m (2H); 2.99 m (2H); 1.27 m (6H). | 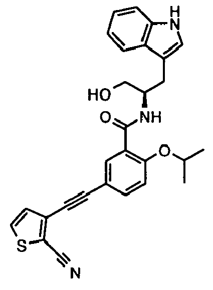 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *3-Bromo-thiophene-2-carbonitrile* | | | |
| **17** | N-[(R)-1-Hydroxymethyl-2-(1H-indol-3-yl)-ethyl]-2-isopropoxy-5-(2,4,6-trimethyl-phenylethynyl)-benzamide; | **8** | **¹H-NMR (DMSO-d₆):** 10.83 s (1H); 8.42 d (*J* = 8.1 Hz, 1H); 8.03 d (*J* = 2.3 Hz, 1H); 7.70 d (*J* = 7.7 Hz, 1H); 7.62 dd (*J* = 2.1 Hz / 8.5, 1H); 7.34 d (*J* = 7.9 Hz, 1H); 7.23 d (*J* = 8.9 Hz, 1H); 7.16 d (*J* = 1.9 Hz, 1H); 7.07 m (1H); 6.97 m (3H); 4.97 m (1H); 4.82 m (1H); 4.26 m(1H); 3.49 m (2H); 2.98 m (2H); 2.51 s (6H); 2.43 s (3H); 1.27 m (6H). | 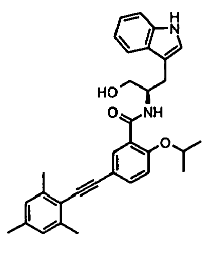 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *2-Bromo-1, 3,5-trimethyl-benzene* | | | |
| **18** | N-[(R)-1-Hydroxymethyl-2-(1H-indol-3-yl)-ethyl]-2-isopropoxy-5-(2,4,5-trimethyl-phenylethynyl)-benzamide; | **8** | **¹H-NMR (DMSO-d₆):** 10.83 s (1H); 8.41 d (*J* = 8.3 Hz, 1H); 8.02 d (*J* = 2.3 Hz, 1H); 7.70 d (*J* = 7.7 Hz, 1H); 7.60 dd (*J* = 8.5 Hz / 2.3 Hz, 1H); 7.34 d (*J* = 7.9 Hz, 1H); 7.29 s (1H); 7.23 d (*J* = 8.9 Hz, 1H); 7.16 d (*J* = 2.1 Hz, 1H); 7.09 s (1H); 7.07 m (1H); 6.98 m (1H); 4.96 m (1H); 4.82 m (1H); 4.25 m (1H); 3.49 m (2H); 2.99 m (2H); 2.39 s (3H); 2.22 s (3H); 2.20 s (3H); 1.27 m (6H). | 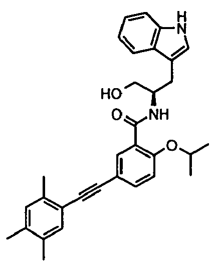 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-2,4,5-trimethyl-benzene* | | | |
| **19** | 5-[4-(2-Hydroxy-ethyl)-phenylethynyl]-N-[(R)-1-hydroxymethyl-2-(1H-indol-3-yl)-ethyl]-2-isopropoxy-benzamide; | **8** | **¹H-NMR (DMSO-d₆):** 10.83 s (1H); 8.39 d (*J* = 8.1 Hz, 1H); 8.02 d (*J* = 2.5 Hz, 1H); 7.69 d (*J* = 7.7 Hz, 1H); 7.62 dd (*J* = 8.5 Hz / 2.3 Hz, 1H); 7.47 d (*J* = 8.1 Hz, 2H); 7.34 d (*J* = 7.9 Hz, 1H); 7.28 d (*J* = 8.1 Hz, 2H); 7.22 d (*J* = 8.9 Hz, 1H); 7.16 d (*J* = 1.8 Hz, 1H); 7.07 m (1H); 6.98 m(1H); 4.96 m (1H); 4.82 m (1H); 4.68 m (1H); 4.25 m (1H); 3.62 m (2H); 3.49 m (2H); 2.98 m (2H); 2.76 m (2H); 1.26 m (6H). | 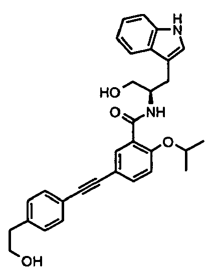 |
| | | | | |
| | *5-Ethynyl-N-[(R)-1-hydroxymethyl-2-(1H-indol-3-yl)-ethyl]-2-isopropoxy*-*benzamide* | | | |
| | | | | |
| | *and* | | | |
| | | | | |
| | *2-(4-Bromo-phenyl)-ethanol* | | | |

### Example 20

### N-[(R)-2-Hydroxy-1-(1H-indol-3-ylmethyl)-2-methyl-propyl]-2-isopropoxy-5-(2-methoxy-phenylethynyl)-benzamide

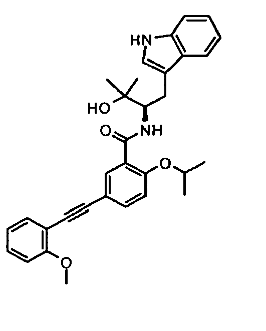

### 20a) 2-Isopropoxy-5-(2-methoxy-phenylethynyl)-benzoic acid

The title compound was prepared in analogy to example 1. **¹H-NMR (DMSO-d₆):** 7.69 d (*J* = 2.3 Hz, 1H); 7.59 dd (*J* = 2.3 Hz / 8.7 Hz, 1H); 7.48 dd (*J* = 1.7 Hz / 7.5 Hz, 1H); 7.38 m (1H); 7.17 d (*J* = 8.9 Hz, 1H); 7.09 d (*J* = 8.1 Hz, 1H); 6.98 td (*J* = 0.9 Hz / 7.5 Hz, 1H); 4.72 m (1H); 3.86 s (3H); 1.30 s (3H); 1.28 s (3H).

### 20b) (R)-3-(1H-Indol-3-yl)-2-[2-isopropoxy-5-(2-methoxy-phenylethynyl)-benzoylamino]-propionic acid methyl ester

A solution of 2-Isopropoxy-5-(2-methoxy-phenylethynyl)-benzoic acid (244 mg, 1 eq.), HATU (299 mg, 1eq.), triethylamine (0.27 ml, 2 eq.) and (D) tryptophanol methyl ester hydrochloride (200 mg, 1 eq.) in DMF (4 ml) was stirred overnight at ambient temperature under argon. The reaction mixture was poured on ammonium chloride / ice, the precipitate was filtered off and washed with water. The title compound was dried in vacuum and used without further purification (356 mg, 89% yield). **¹H-NMR (DMSO-d₆):** 10.93 s (1H); 8.64 d (*J* = 7.5 Hz, 1H); 8.02 d (*J* = 2.3 Hz, 1H); 7.61 dd (*J* = 8.7 Hz / 2.5 Hz, 1H); 7.49 dd (*J* = 7.5 Hz / 1.7 Hz, 1H); 7.41 d (*J* = 8.3 Hz, 1H); 7.34 d (*J* = 8.1 Hz, 1H); 7.20 d (*J* = 8.9 Hz, 1H); 7.11 m (2H); 6.99 m (1H); 6.92 m (1H); 4.98 m (1H); 4.73 m (1H); 3.88 s (3H); 3.67 s (3H); 3.30 m (2H); 1.10 d (*J* = 6.0 Hz, 1H); 0.98 d (*J* = 6.0 Hz, 1H).

### 20c)N-[(R)-2-Hydroxy-1-(1H-indol-3-ylmethyl)-2-methyl-propyl]-2-isopropoxy-5-(2-methoxy-phenylethynyl)-benzamide

(R)-3-(1H-Indol-3-yl)-2-[2-isopropoxy-5-(2-methoxy-phenylethynyl)-benzoylamino]-propionic acid methyl ester (60 mg, 1eq.) was dissolved in THF (3 ml) and cooled to -5°C. A solution of MeMgBr (3M in ether, 0.59 ml) was added slowly and the reaction was allowed to warm to ambient temperature over two hours. The reaction was terminated by addition of saturated ammonium chloride solution (5 ml), the reaction was stirred for 20 min and the water phase was extracted with MTBE (3x20 ml). The combined organic layers were washed with water (2x 20 ml), dried over magnesium sulphate, the solvent was removed and the product was dried in vacuum.The title compound was obtained in 68% yield (41 mg) after flash chromatography.

**¹H-NMR (DMSO-d₆):** 10.63 s (1H); 8.16 d (*J* = 9.9 Hz, 1H); 7.63 d (*J* = 2.3 Hz, 1H); 7.53 d (*J* = 7.8 Hz, 1H); 7.49 dd (*J* = 2.3 Hz / 8.6 Hz, 1H); 7.42 dd (*J* = 1.8 Hz / 9.4 Hz, 1H); 7.33 td (*J* = 1.8 Hz / 8.6 Hz, 1H); 7.25 d (*J* = 8.1 Hz, 1H); 7.14 d (*J* = 8.8 Hz, 1H); 7.02 m (3H); 6.92 m (2H); 4.79 m (1H); 4.65 s (1H); 4.22 m (1H); 3.81 s (3H); 3.22 m (1H); 2.69 m (1H); 1.36 s (3H); 1.26 m (6H); 1.14 s (3H).

### Example 21

### N-[2-(5-Fluoro-1H-indol-3-yl)-1-hydroxymethyl-ethyl]-2-isopropoxy-5-phenylethynyl-benzamide

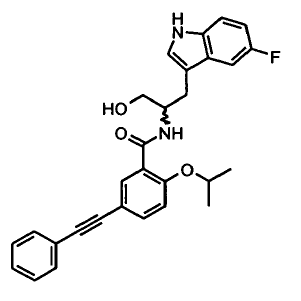

### 21 a) 2-Isopropoxy-5-phenylethynyl-benzoic acid

The title compound was prepared in analogy to example 1. **¹H-NMR (CDCl₃):** 8.38 d (J = 2.3 Hz, 1H); 7.67 dd (*J* = 2.3 Hz / 8.7 Hz, 1H); 7.50 m (2H); 7.36 m (2H); 7.34 d (*J* = 2.6 Hz, 1H); 7.03 d (*J* = 8.7 Hz, 1H); 4.89 m (1H); 1.52 s (3H); 1.50 s (3H).

### 21 b)N-[2-(5-Fluoro-1H-indol-3-yl)-1-hydroxymethyl-ethyl]-2-isopropoxy-5-phenylethynylbenzamide

A solution of 2-Isopropoxy-5-phenylethynyl-benzoic acid (54 mg, 1 eq.), HOBt (29 mg, 1 eq.), EDC (37 mg, 1 eq.) and 5-Fluor-(D/L)-tryptophanol (40 mg, 1 eq.) in DMF (3 ml) were stirred at ambient temperature overnight. The reaction was quenched by addition of saturated aqueous sodium carbonate solution (50 ml) and the resulting precipitate was filtered off and washed with water. After flash chromatography 67 mg of the title compound was obtained. **¹H-NMR (DMSO-d₆):** 10.90 s (1H); 8.34 d (*J* = 8.1 Hz, 1H); 7.99 d (*J* = 2.5 Hz, 1H); 7.58 dd (*J* = 2.5 Hz / 8.6 Hz, 1H); 7.51 m (2H); 7.38 m (4H); 7.28 m (1H); 7.19 m (2H); 6.86 td (*J* = 2.5 Hz / 9.4 Hz, 1H); 4.94 m (1H); 4.78 m (1H); 4.16 m (1H); 3.45 m (2H); 2.91 m (2H); 1.23 m (6H).

The following compounds were obtained in analogy to the preparation methods described in detail:

| **Ex.** | **Product; *reagents*** | **Method analogous to** | **ESI-MS** | **Structure** |
|---|---|---|---|---|
| **22** | N-[2-(5-Fluoro-1H-indol-3-yl)-1- hydroxymethyl-ethyl]-2-isopropoxy-5-(2-methoxy-phenylethynyl)-benzamide; | **21** | Molecular peak (ESI, M+1): 501 | 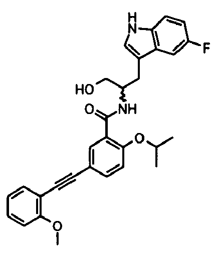 |
| | | | | |
| | *2-Isopropoxy-5-(2-methoxy-phenylethynyl)-benzoic acid* | | | |
| | | | | |
| | *and* | | | |
| | | | | |
| | *2-Amino-3-(5-fluoro- 1H-indol-3-yl)-propan-1-ol* | | | |

### Example 23

### N-[(RS)-2-(5,6-Difluoro-1H-indol-3-yl)-1-hydroxymethyl-ethyl]-2-isopropoxy-5-(2-methoxy-phenylethynyl)-benzamide

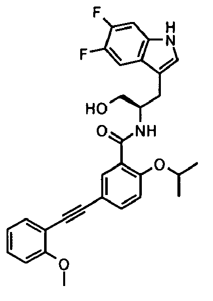

### 23a) 5,6-Difluoro-1H-indole-3-carbaldehyde

Phosphoryl cloride (22.03 g, 143.67 mmol) was added drop wise to an ice-cold N,N-dimethylformamide (DMF) (19.10 g, 261.22 mmol) and the mixture was stirred for 0.5 h at 0-5 °C and for 1 h at room temperature. Then the mixture was cooled to 0-5 °C and a solution of 5,6-difluoro-1*H*-indole (20.00 g, 130.61 mmol) in DMF (20 g) was added drop wise. The resulting mixture was stirred for 0.5 h at 0-5 °C and for 15 h at room temperature. The mixture was poured onto chopped ice (200 g) and alkalinized with NaOH to pH=10. The crystalline product was filtered off, washed with water and dried to provide the title comound (22.72 g, 96.01 %).

### 23b) (5,6-Difluoro-1H-indol-3-ylmethyl)-diethyl-amine

Sodium triacetoxiborohydride (26.33 g, 124.21 mmol) was added portion wise to the solution of 5,6-difluoro-1*H*-indole-3-carbaldehyde (15.00 g, 82.81 mmol) and diethyl amine (6.66 g, 91.09 mmol) in abs. CH₂Cl₂ (300 ml) with two drops of TFA and this mixture was stirred for 24 hours at room temperature, evaporated to dryness and treated with 10 % NaHCO₃ (100 ml). Organic layer was extracted with EtOAc, dried over anhydrous sodium sulfate, filtered and concentrated under reduced pressure. The residue was purified by flash chromatography (SiO₂, CHCl₃ : MEOH = 19 : 1) to provide the title compound (13.50 g, 68.42 %).

### 23c) 3-(5,6-Difluoro-1H-indol-3-yl)-2-nitro-propionic acid ethyl ester

The mixture of gramine (8.000 g, 33.57 mmol) and ethyl 2-nitroacetat (8.937 g, 67.15 mmol) was heated to 90 - 100 °C in abs. toluene for 4 hours while argon was passed through the mixture. The mixture was concentrated under reduced pressure and purified by flash flash chromatography (SiO₂, CHCl₃ MeOH = 19 : 1) to provide an oil (11.70 g) that is the mixture of the title compound with ethyl 2-nitroacetat in ratio 1 : 2.2.

### 23d) 2-Amino-3-(5,6-difluoro-1H-indol-3-yl)-propionic acid ethyl ester

The mixture from **step 23c** (11.70 g) was refluxed with ammonium format (9.895 g, 156.9 mmol) and Pd (4.174 g of 10 % on carbon) in 300 ml of ethanol for 15 h. The mixture was filtered, concentrated under reduced pressure, treated with water (100 ml) and extracted with EtOAc. The organic layer was dried over sodium sulfate, filtered and concentrated under reduced pressure. The residue was purified by flash chromatography (SiO₂, CHCl₃ : MeOH = 19 : 1) to provide the mixture the product and ethyl 2-aminoacetate. Through the solution of this mixture in abs. ether gaseous HCl was passed. The obtained crystals were filterer off and recrystallized from EtOH to provide the title compound (2.7 g) as hydrochloride. 2-Amino-3-(5,6-difluoro-1H-indol-3-yl)-propionic acid ethyl ester, HCl salt

### 23e) 3-(5,6-Difluoro-1H-indol-3-yl)-2-[2-isopropoxy-5-(2-methoxy-phenylethynyl)-benzoylamino]-propionic acid ethyl ester

A solution of 2-Amino-3-(5,6-difluoro-1H-indol-3-yl)-propionic acid ethyl ester HCl salt (100 mg), HOBt (57 mg), EDC (71 mg) and 2-Isopropoxy-5-(2-methoxy-phenylethynyl)-benzoic acid (116 mg) in DMF (4 ml) was stirred at ambient temperature overnight. The reaction mixture was poured on aqueous saturated bicarbonate solution (50 ml), the resulting precipitate was filtered off and washed with water. 111 mg of the title compound was obtained after flash chromatography. **¹H-NMR (DMSO-d₆):** 11.07 s (1H); 8.60 d (*J* = 7.6 Hz, 1H); 7.97 d (*J* = 2.3 Hz, 1H); 7.57 dd (*J* = 8.6 Hz / 2.3 Hz, 1H); 7.45 dd (*J* = 7.6 Hz / 1.8 Hz, 1H); 7.37-7.26 m (3H); 7.17 d (*J* = 8.8 Hz, 1H); 7.15 d (*J* = 2.3 Hz, 1H); 7.06 d (*J* = 8.1 Hz, 1H); 6.94 td (*J* = 7.6 Hz / 1.1 Hz, 1H); 4.87 m (1H); 4.72 m (1H); 4.07 m (2H); 3.84 s (3H); 3.23 m (2H); 1.12 m (6H); 0.97 m (3H).

### 23f) N-[(RS)-2-(5,6-Difluoro-1H-indol-3-yl)-1-hydroxymethyl-ethyl]-2-isopropoxy-5-(2-methoxy-phenylethynyl)-benzamide

To a solution of 3-(5,6-Difluoro-1H-indol-3-yl)-2-[2-isopropoxy-5-(2-methoxy-phenylethynyl)-benzoylamino]-propionic acid ethyl ester (111 mg) in THF (3 ml) was added a LiBH₄ (2M solution in THF, 0.15 ml) at 0°C. The reaction mixture was allowed to warm to ambient temperature and stirred overnight. The reaction was cooled to 0°C and neutralized with HCI (1N), water was added and the mixture was extracted with ethyl acetate (3x 30 ml). The combined organic layers were dried over magnesium sulphate and the solvent removed. The title compound was obtained in 13 mg after flash chromatography. **¹H-NMR (DMSO-d₆):** 10.97 s (1H); 8.36 d (*J* = 8.1 Hz, 1H); 7.95 d (*J* = 2.3 Hz, 1H); 7.60 dd (*J* = 8.1 Hz / 11.2 Hz, 1H); 7.55 dd (*J* = 8.6 Hz / 2.5 Hz, 1H); 7.44 d (*J* = 7.6 Hz / 1.8 Hz, 1H); 7.33 m (2H); 7.19 m (2H); 7.04 d (*J* = 8.1 Hz, 1H); 6.94 td (*J* = 7.6 Hz / 0.8 Hz, 1H); 4.96 m (1H); 4.78 m (1H); 4.14 m (1H); 3.83 s (3H); 3.44 m (2H); 2.92 m (2H); 1.23 m (6H).

### Example 24

### N-[(R)-2-(1-Ethyl-1H-indol-3-yl)-1-hydroxymethyl-ethyl]-2-isopropoxy-5-(2-methoxy-phenylethynyl)-benzamide

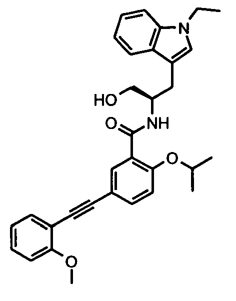

### 24a) Methyl (R)-2-tert-butoxycarbonylamino-3-(1H-indol-3yl)-propionate

15.72 mmol (2.18 ml) of triethylamine were added dropwise to a solution of 3.93 mmol (1g) of D-tryptophan methyl ester hydrochloride in 35 ml of dichloromethane with stirring and then 7.85 mmol (1.71 g) of di-tert-butyl dicarbonate, dissolved in 5 ml of dichloromethane, were added, followed by 0.39 mmol (48 mg) of dimethylaminopyridine. The mixture was stirred at room temperature for about 1.5 h. Then 25 ml of 10% strength sodium bisulphite solution were added to the reaction mixture and stirred for 15 minutes. After phase separation, the aqueous phase was extracted with dichloromethane. The resulting organic phase was dried over magnesium sulphate, filtered and concentrated in vacuo. Purification by chromatography on silica gel with the eluent cyclohexane/ethyl acetate affords 800 mg of the compound as a white solid. **¹H-NMR (400 MHz, DMSO-d₆):** δ [ppm] = 10.84 s (1H, NH); 7.45 d (*J* = 7.8 Hz, 1H, aryl) 7.32 d (1H, aryl); 7.14 s (1H, aryl); 7.05 t (*J* = 7.4 Hz, 1H, aryl); 6.97 t (*J* = 7.9 Hz, 1H, aryl); 4.20 m (1H, CH); 3.59 s (3H, OCH₃); 3.08 dd (*J* = 14.4 Hz / 5.8 Hz, 1H, CH); 3.00 dd (*J* = 14.4 Hz / 8.2 Hz, 1H, CH); 1.33 s (9H, CH₃).

### 24b) Methyl (R)-2-tert-butoxycarbonylamino-3-(1-ethyl-1H-indol-3-yl)propionate

3.27 mmol (183 mg) of potassium hydroxid powder were added in portions to a stirred solution of 2.51 mmol (800 mg) of the protected amino acid prepared in a), in 8 ml of DMSO, slightly cooling in water. This mixture was stirred for 5 minutes and then 3.27 mmol (0.26 ml) of ethyl iodide, dissolved in 2 ml of DMSO, were added dropwise. Stirring was continued at room temperature for 2 hours, and the reaction mixture was then added to saturated aqueous ammonium chloride solution and extracted with ethyl acetate. The resulting organic phase was dried over magnesium sulphate, filtered and concentrated in vacuo with the addition of toluene. 871 mg of the target compound are obtained. **¹H-NMR (400 MHz, DMSO-d₆):** δ [ppm] = 7.48 d (*J* = 7.8 Hz, 1H, aryl); 7.41 d (*J* = 7.8 Hz, 1H, aryl); 7.23 d (*J* = 7.4 Hz, 1H, aryl); 7.11 t (*J* = 7.6 Hz, 1H, aryl); 7.00 t (J = 7.8 Hz, 1H, aryl); 4.20 m (1H, CH); 4.19 q (*J* = 7.0 Hz, 2H, CH₂); 3.07 dd (14.3 Hz / 5.3 Hz, 1H, CH); 3.01 dd (*J* = 14.3 Hz / 8.2 Hz, 1H, CH); 1.33 s (9H, CH₃); 1.3 t (*J* = 7.0 Hz, 3H, CH₃).

### 24c) Methyl (R)-2-amino-3-(1-ethyl-1H-indol-3-yl)propionate

2.48 mmol (860 mg) of the compound prepared in b) were dissolved in 10 ml of dichloromethane and then 24.8 mmol (1.91 ml) of trifluoroacetic acid were added dropwise at room temperature. After 1 hour, 20 ml of saturated sodium bicarbonate solution were cautiously added dropwise to the mixture until the neutral point was reached. After phase separation, the aqueous phase was extracted with dichloromethane. The resulting organic phase was dried over magnesium sulphate, filtered and concentrated in vacuo. 588 mg of the product are obtained. **¹H-NMR (400 MHz, DMSO-d₆):** δ [ppm] = 7.47 d (*J* = 7.8 Hz, 1H, aryl); 7.39 d (*J* = 7.8 Hz, 1H, aryl); 7.15 s (1H, aryl); 7.09 t (*J* = 7.5 Hz, 1H, aryl); 6.98 t (*J* = 7.8 Hz, 1H, aryl); 4.14 q (*J* = 7.0 Hz, 2H, CH₂); 3.57 m (1H, CH); 3.54 s (3H, OCH₃); 2.98 dd (*J* = 14.2 Hz / 5.4 Hz, 1H, CH); 2.93 dd (*J* = 14.2 Hz / 8.4 Hz, 1H, CH); 1.79 s (2H, NH₂,); 1.31 t (*J* = 7.0 Hz, 3H, CH₃).

### 24d) (R)-3-(1-Ethyl-1H-indol-3-yl)-2-[2-isopropoxy-5-(2-methoxy-phenylethyn yl)-benzoylamino]-propionic acid methyl ester

A solution of Methyl (R)-2-amino-3-(1-ethyl-1H-indol-3-yl)propionate (120 mg), 2-Isopropoxy-5-(2-methoxy-phenylethynyl)-benzoic acid (151 mg), HATU (185 mg), Hünig base (0.17 ml) in DMF (10 ml) was stirred at ambient temperature overnight. The reaction mixture was poured on ice / ammonium chloride solution and the precipitate filtered off. The title compound was isolated in 60 % yield (157 mg) after flash chromatography. **¹H-NMR (DMSO-d₆):** 8.60 d (*J* = 7.6 Hz, 1H); 7.96 d (*J* = 2.5 Hz, 1H); 7.56 dd (*J* = 8.6 Hz / 2.5 Hz, 1H); 7.45 dd (*J* = 7.6 Hz / 1.5 Hz, 1H); 7.37 m (3H); 7.14 d (*J* = 8.8 Hz, 1H); 7.10 s (1H); 7.05 m (2H); 6.94 m (1H); 6.89 m (1H); 4.90 m (1H); 4.66 m (1H); 4.10 m (2H); 3.83 s (3H); 3.64 s (3H); 3.24 m (2H); 1.25 m (3H); 1.06 d (*J* = 6.1 Hz, 1H); 0.87 d (*J* = 6.1 Hz, 1H).

### 24e) N-[(R)-2-(1-Ethyl-1H-indol-3-yl)-1-hydroxymethyl-ethyl]-2-isopropoxy-5-(2-methoxy-phenylethynyl)-benzamide

To a solution of (R)-3-(1-Ethyl-1H-indol-3-yl)-2-[2-isopropoxy-5-(2-methoxy-phenylethyn yl)-benzoylamino]-propionic acid methyl ester (150 mg) in THF (10 ml) was added lithium borohydride (2 M solution in THF, 0.21 ml) at -10°C. The reaction was allowed to warm to ambient temperature and stirred overnight. The reaction was cooled to 0°C, quenched with water (4 ml), neutralized with HCl (1N) and extracted with MTBE. The solvent was removed and the product purified via flash chromatography to yield 155 mg of the title compound. **¹H-NMR (DMSO-d₆):** 8.35 d (*J* = 8.1 Hz, 1H); 7.96 d (*J* = 2.3 Hz, 1H); 7.66 d (*J* = 8.1 Hz, 1H); 7.56 dd (*J* = 2.5 Hz / 8.6 Hz, 1H); 7.43 m (1H); 7.36 m (3H); 7.16 m (2H); 7.04 m (2H); 6.94 m (2H); 4.92 m (1H); 4.77 m (1H); 4.19 m (1H); 4.11 m (2H); 3.83 s (3H); 3.46 m (2H); 2.94 m (2H); 1.28 m (9H).

The following compounds were obtained in analogy to the preparation methods described in detail:

| **Ex.** | **Product; *reagents*** | **Method analogous to** | **¹H-NMR (400 MHz) δ [ppm]** | **Structure** |
|---|---|---|---|---|
| **25** | N-[(R)-2-(1-Ethyl-1H-indol-3-yl)-1-hydroxymethyl-ethyl]-2-isopropoxy-5-phenylethynyl-benzamide; | **24** | **¹H-NMR (DMSO-d₆):** 8.34 d (*J* = 8.1 Hz, 1H); 7.98 d (*J* = 2.3 Hz, 1H); 7.66 d (*J* = 7.8 Hz, 1H); 7.59 dd (*J* = 8.6 Hz / 2.3 Hz, 1H); 7.51 m (2H); 7.38 m (4H); 7.18 d (*J* = 8.8 Hz, 1H); 7.16 s (1H); 7.08 m (1H); 6.96 m (1H); 4.92 m (1H); 4.77 m (1H); 4.19 m (1H); 4.11 m (2H); 3.45 m (2H); 2.93 m (2H); 1.24 m (9H). | 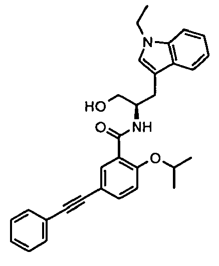 |
| | | | | |
| | *(R)-3-(1-Ethyl-1H-indol-3-yl)-2-[2-isopropoxy-5-(phenyl-ethynyl)-benzoylamino]-propionic acid methyl ester* | | | |

### Example 26

### 5-(3-Amino-1H-indazol-4-ylethynyl)-N-[(R)-2-hydroxy-1-(1H-indol-3-ylmethyl)-ethyl]-2-isopropoxy-benzamide

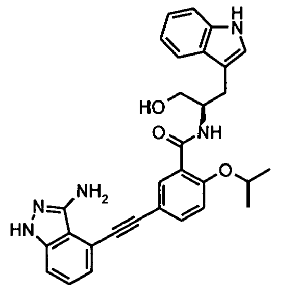

### 26a) 4-lodo-1H-indazol-3-ylamine

To the pale yellow stirred solution of 2-Fluoro-6-iodo-benzonitrile in PrOH, in a sealed Radley's Caroussel tube, was added hydrazine hydrate by syringe. The reaction was stirred at 100 °C (heating block temperature) for 4 hours. The reaction was cooled and diluted with water and allowed to stand overnight. The solution was extracted (3 x) with dichloromethane and the combined organic layers dried through a Whatman phase separator and concentrated in vacuo. The crystalline residue was suspended in hexane, filtered, washed with hexane and dried to give the title compound as fine pale yellow needles, 0.591 g, mp 152.7 °C. The material was used without further purification.

### 26a) 5-(3-Amino-1H-indazol-4-ylethynyl)-N-[(R)-2-hydroxy-1-(1H-indol-3-ylmethyl)-ethyl]-2-isopropoxy-benzamide

The title compound was prepared in analogy to example 8. **¹H-NMR (DMSO-d₆):** 11.74 s (1H); 10.78 s (1H); 8.35 d (*J* = 7.9 Hz, 1H); 8.28 s (1H); 8.05 d (*J* = 2.3 Hz, 1H); 7.64 m (2H); 7.24 m (4H); 7.08 m (5H); 5.10 m (1H); 4.91 m (1H); 4.80 m (1H); 4.21 m (1H); 3.44 m (2H); 2.90 m (2H); 1.23 m (6H).

### Example 27

### N-[(R)-1-Hydroxymethyl-2-(1H-indol-3-yl)-ethyl]-5(4-hydroxy-phenylethynyl)-2-isopropoxy-benzamide

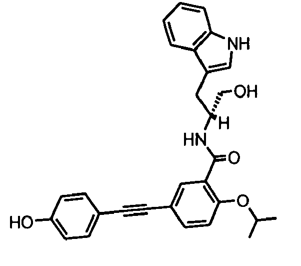

To a solution of 4-Bromo phenol (0.2 M in THF, 1.5 ml) was added a solution of 5-Ethynyl-N-[(R)-1-hydroxymethyl-2-(1H-indol-3-yl)-ethyl]-2-isopropoxy-benzamide (0.2 M in THF, 1ml), a solution of PdCl₂(PPh₃)₂ (0.012 M in THF, 550µl) and a solution of TBAF (1 M in THF, 600µl) at ambient temperature. The reaction mixture was heated in a focussed microwave apparatus at 80°C for 30 minutes at 600W in a sealed tube. The solvent was evaporated under reduced pressure and the product was purified by HPLC to yield the title compound in 22% yield.

HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75')
Molecular peak (ESI, M+1): 470
Retention time: 3.96 min.

The following compounds were obtained in analogy to the preparation methods described in detail:

| **Ex.** | **Product; *reagents*** | **Method analogous to** | **HPLC-MS** | **Structure** |
|---|---|---|---|---|
| **28** | N-[(R)-1-Hydroxymethyl-2-(1H-indol-3-yl)-ethyl]-2-isopropoxy-5-(6-methoxy-naphthalen-2-ylethynyl)-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1 % to 99% (5') to 99% (1') to 1% (0.25') to 1% (1.75') | 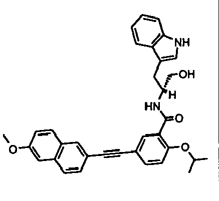 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *2-Bromo-6-methoxy-naphthalene* | | Molecular peak (ESI, M+1): 534 | |
| | | | Retention time: 4.7 min. | |
| **29** | N-[(R)-1-Hydroxymethyl-2-(1H-indol-3-yl)-ethyl]-2-isopropoxy-5-m-tolylethynyl-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 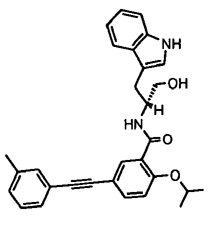 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-3-methyl-benzene* | | Molecular peak (ESI, M+1): 468 | |
| | | | Retention time: 4.55 min. | |
| **30** | 5-(3-Fluoro-4-methoxy-phenylethynyl)-N-[(R)-2-hydroxy-1-(1H-indol-3-ylmethyl)-ethyl]-2-isopropoxy-benzamide; | 27 | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 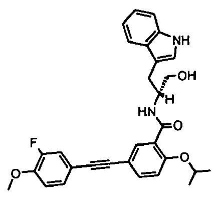 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | Molecular peak (ESI, M+1): 502 | |
| | | | | |
| | *4-Bromo-2-fluoro-1-methoxy-benzene* | | Retention time: 4.34 min. | |
| **31** | N-[(R)-1-Hydroxymethyl-2-(1H-indol-3-yl)-ethyl]-5-(3-hydroxy-6-methyl-pyridin-2-ylethynyl)-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 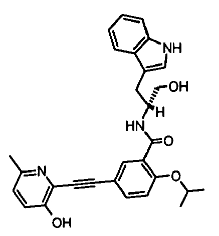 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *2-Bromo-6-methyl-pyridin-3-ol* | | Molecular peak (ESI, M+1): 485 | |
| | | | Retention time: 2.96 min. | |
| **32** | 4-{3-[(R)-1-Hydroxymethyl-2-(1H-indol-3-yl)-ethylcarbamoyl]-4-isopropoxy-phenylethynyl}-benzoic acid methyl ester; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 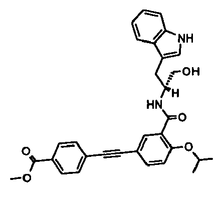 |
| | | | | |
| | *5-Ethynyl-N-[(R)-1-hydroxymefhyl-2-(1H-indol-3-yl)-ethyl]-2-isopropoxy*-*benzamide* | | | |
| | | | | |
| | *and* | | | |
| | | | | |
| | *4-Bromo-benzoic acid methyl ester* | | Molecular peak (ESI, M+1): 512 | |
| | | | Retention time: 4.36 min. | |
| **33** | 5-{3-[(R)-1-Hydroxymethyl-2-(1H-indol-3-yl)-ethylcarbamoyl]-4-isopropoxy-phenylethynyl}-nicotinic acid methyl ester; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 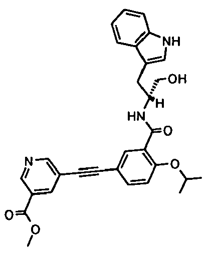 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *5-Bromo-nicotinic acid methyl ester* | | Molecular peak (ESI, M+1): 513 | |
| | | | Retention time: 3.88 min. | |
| **34** | 5-{3-[(R)-2-Hydroxy-1-(1H-indol-3-ylmethyl)-ethylcarbamoyl]-4-isopropoxy-phenylethynyl}-thiophene-2-carboxylic acid ethyl ester; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 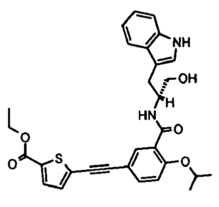 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | Molecular peak (ESI, M+1): 532 | |
| | | | | |
| | *5-Bromo-thiophene-2-carboxylic acid ethyl ester* | | Retention time: 4.53 min. | |
| **35** | N-[(R)-1-Hydroxymethyl-2-(1H-indol-3-yl)-ethyl]-5-(6-hydroxy-naphthalen-2-ylethynyl)-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 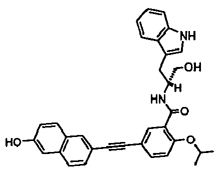 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *6-Bromo-naphthalen-2-ol* | | Molecular peak (ESI, M+1): 520 | |
| | | | Retention time: 4.05 min. | |
| **36** | 3-{3-[(R)-1-Hydroxymethyl-2-(1H-indol-3-yl)-ethylcarbamoyl]-4-isopropoxy-phenylethynyl}-benzoic acid; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 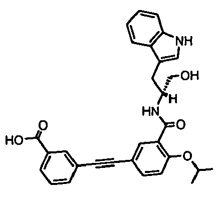 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *3-Bromo-benzoic acid* | | Molecular peak (ESI, M+1): 498 | |
| | | | Retention time:3.79 min. | |
| **37** | N-[(R)-1-Hydroxymethyl-2-(1H-indol-3-yl)-ethyl]-5-(2-hydroxymethyl-phenylethynyl)-2-isopropoxy-benzamide; | **27** | HPLC purification: Col-umn X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 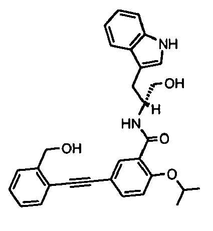 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *(2-Bromo-phenyl)-methanol* | | Molecular peak (ESI, M+1): 484 | |
| | | | Retention time: 3.87 min. | |
| **38** | 5-(5-Fluoro-2-methoxy-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 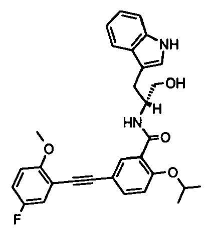 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | Molecular peak (ESI, M+1): 502 | |
| | | | | |
| | *2-Bromo-4-fluoro-1-methoxy-benzene* | | Retention time: 4.29 min. | |
| **39** | 5-(3-Cyano-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 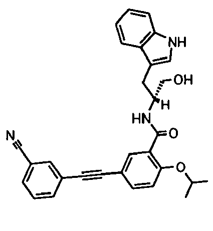 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | *3-Bromo-benzonitrile* | | Molecular peak (ESI, M+1): 479 | |
| | | | Retention time: 4.18 min. | |
| **40** | 5-(3,5-Dimethyl-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 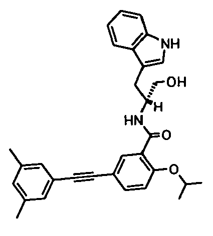 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-3,5-dimethyl-benzene* | | Molecular peak (ESI, M+1): 482 | |
| | | | Retention time: 4.84 min. | |
| **41** | 5-(2,6-Difluoro-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 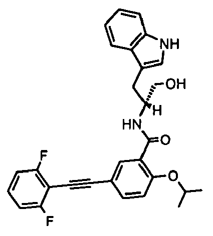 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *2-Bromo-1, 3-difluoro-benzene* | | Molecular peak (ESI, M+1): 490 | |
| | | | Retention time: 4.34 min. | |
| **42** | 5-(3,5-Difluoro-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow | 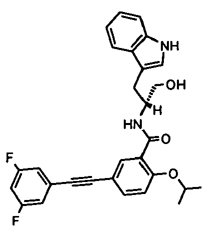 |
| | | | | |
| | *5-Ethynyl-N-[(R)-1-hydroxymethyl-2-(1H-indol-3-yl)-ethyl]-2-isopropoxy-benzamide* | | rate 2 ml/min; eluents A: 0.1% TFA in H₂O, B 0.1% TFA in ACN; gradient in each case based on B: 1 % to 99% (5') to 99% (1') | |
| | | | | |
| | *and* | | to 1% (0.25') to 1% (1.75') | |
| | | | | |
| | *1-Bromo-3,5-difluoro-benzene* | | Molecular peak (ESI, M+1): 490 | |
| | | | Retention time: 4.56 min. | |
| **43** | N-[(R)-2-Hydroxy-1-(1H-indol-3-ylmethyl)-ethyl]-2-isopropoxy-5-(3-trifluoromethyl-phenylethynyl)-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') Molecular peak (ESI, M+1): 522 Retention time: 4.73 min. | 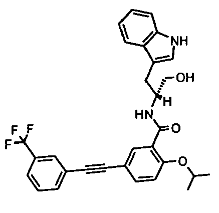 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-3-trifluoromethyl-benzene* | | | |
| | | | | |
| **44** | 5-(2-Chloro-4-hydroxy-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 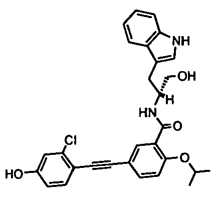 |
| | | | | |
| | *5-Ethynyl-N-[(R)-1-hydroxymethyl-2-(1H-indol-3-yl)-ethyl]-2-isopropoxy benzamide* | | | |
| | | | | |
| | *and* | | | |
| | | | | |
| | *4-Bromo-3-chloro-phenol* | | Molecular peak (ESI, M+1): 504 | |
| | | | Retention time: 3.95 min. | |
| **45** | N-[(R)-1-Hydroxymethyl-2-(1H-indol-3-yl)-ethyl]-2-isopropoxy-5-(4-methoxy-phenylethynyl)-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 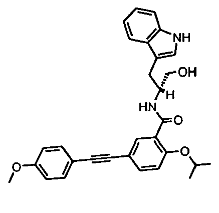 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-4-methoxy-benzene* | | Molecular peak (ESI, M+1): 484 | |
| | | | Retention time: 4.33 min. | |
| **46** | N-[(R)-1-Hydroxymethyl-2-(1H-indol-3-yl)-ethyl]-2-isopropoxy-5-pyrimidin-5-ylethynyl-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 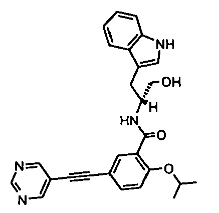 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *5-Bromo-pyrimidine* | | Molecular peak (ESI, M+1): 456 | |
| | | | Retention time: 3.51 min. | |
| **47** | N-[(R)-1-Hydroxymethyl-2-(1H-indol-3-yl)-ethyl]-5-(3-hydroxy-phenylethynyl)-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1 % (0.25') to 1% (1.75') | 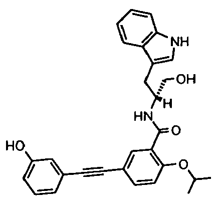 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *3-Bromo-phenol* | | Molecular peak (ESI, M+1): 470 | |
| | | | Retention time: 3.84 min. | |
| **48** | 5-(2, 5-Dimethyl-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 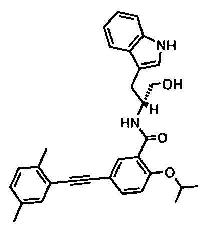 |
| | | | | |
| | *5-Ethynyl-N-[(R)-1-hydroxymethyl-2-(1H-indol-3-yl)-ethyl]-2-isopropoxy benzamide* | | | |
| | | | | |
| | *and* | | | |
| | | | | |
| | *2-Bromo- 1, 4-dimethyl-benzene* | | Molecular peak (ESI, M+1): 482 | |
| | | | Retention time: 4.81 min. | |
| **49** | 5-(3,4-Dichloro-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 mi/min; eluents A: 0.1% TFA in H₂O, B 0.1% TFA in ACN; gradient in each case based on B: 1% to 99% (5') to 99% (1') to 1% (0.25') to 1% (1.75') | 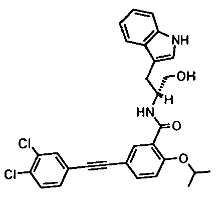 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *4-Bromo-1,2-dichloro-benzene* | | Molecular peak (ESI, M+1): 522 | |
| | | | Retention time: 5 min. | |
| **50** | 5-Benzo[1,3]dioxol-5-ylethynyl-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 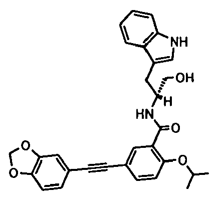 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *5-Bromo-benzo[1, 3]dioxole* | | Molecular peak (ESI, M+1): 498 | |
| | | | Retention time: 4.24 min. | |
| **51** | 5-(2-Cyano-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1 % (0.25') to 1% (1.75') | 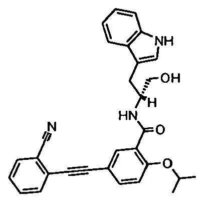 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *2-Bromo-benzonitrile* | | Molecular peak (ESI, M+1): 479 | |
| | | | Retention time: 4.16 min. | |
| **52** | N-[(R)-1-Hydroxymethyl-2-(1H-indol-3-yl)-ethyl]-2-isopropoxy-5-naphthalen-2-ylethynyl-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 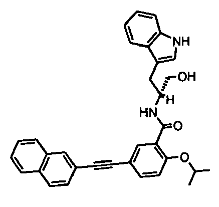 |
| | | | | |
| | *5-Ethynyl-N-[(R)-1-hydroxymethyl-2-(1H-indol-3-yl)-ethyl]-2-isopropoxy*-*benzamide* | | | |
| | | | | |
| | *and* | | | |
| | | | | |
| | *2-Bromo-naphthalene* | | Molecular peak (ESI, M+1): 504 | |
| | | | Retention time: 4.79 min. | |
| **53** | 5-(4-Fluoro-2-methyl-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1 % (0.25') to 1% (1.75') | 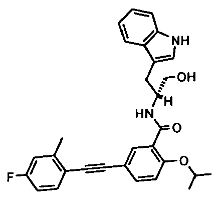 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-4-fluoro-2-methyl-benzene* | | Molecular peak (ESI, M+1): 486 | |
| | | | Retention time: 4.55 min. | |
| **54** | N-[(R)-1-Hydroxymethyl-2-(1H-indol-3-yl)-ethyl]-2-isopropoxy-5-naphthalen-1-ylethynyl-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') | 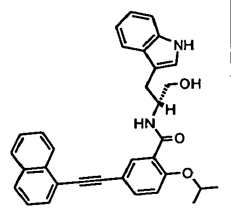 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | to 1% (0.25') to 1% (1.75') | |
| | | | | |
| | *1-Bromo-naphthalene* | | Molecular peak (ESI, M+1): 504 | |
| | | | Retention time: 4.76 min. | |
| **55** | 5-(2,4-Dimethyl-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 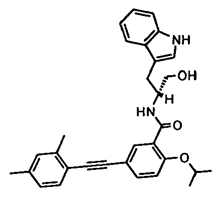 |
| | | | | |
| | *5-Ethynyl-N-[(R)-1-hydroxymethyl-2-(1H-indol-3-yl)-ethyl]-2-isopropoxy-benzamide* | | | |
| | *and* | | | |
| | *1-Bromo-2,4-dimethyl-benzene* | | Molecular peak (ESI, M+1): 482 | |
| | | | Retention time: 4.78 min. | |
| **56** | 5-(2-Fluoro-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 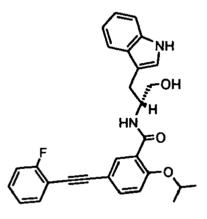 |
| | *5-Ethynyl-N-[(R)-1-hydroxymethyl-2-(1H-indol-3-yl)-ethyl]-2-isopropoxy benzamide* | | | |
| | | | | |
| | *and* | | | |
| | | | | |
| | *1-Bromo-2-fluoro-benzene* | | Molecular peak (ESI, M+1): 472 | |
| | | | Retention time: 4.34 min. | |
| **57** | N-[(R)-1-Hydroxymethyl-2-(1H-indol-3-yl)-ethyl]-2-isopropoxy-5-(3-methoxy-phenylethynyl)-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 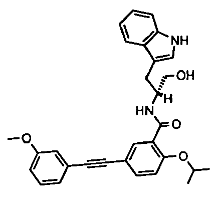 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-Bromo-3-methoxy-benzene* | | Molecular peak (ESI, M+1): 484 | |
| | | | Retention time: 4.36 min. | |
| **58** | (2-{3-[(R)-1-Hydroxymethyl-2-(1H-indol-3-yl)-ethylcarbamoyl]-4-isopropoxy-phenylethynyl}-phenyl)-acetic acid; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 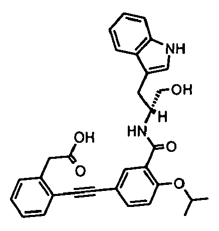 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *(2-Bromo-phenyl)-acetic acid* | | Molecular peak (ESI, M+1): 512 | |
| | | | Retention time: 3.91 min. | |
| **59** | 5-(4-Hydroxy-biphenyl-3-ylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1 % (0.25') to 1% (1.75') | 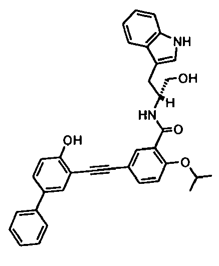 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *3-Bromo-biphenyl-4-ol* | | Molecular peak (ESI, M+1): 546 | |
| | | | Retention time: 4.99 min. | |
| **60** | 5-(3-Chloro-4-cyano-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 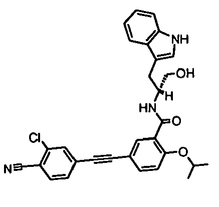 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | Molecular peak (ESI, M+1): 513 | |
| | | | | |
| | *4-Bromo-2-chloro-benzonitrile* | | | |
| | | | Retention time: 4.45min. | |
| **61** | 5-(3-Acetyl-phenylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 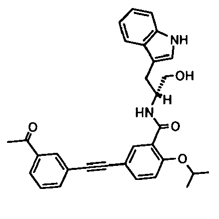 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-(3-Bromo-phenyl)-ethanone* | | Molecular peak (ESI, M+1): 496 | |
| | | | Retention time: 4.14 min. | |
| **62** | 5-(3, 5-Dichloro-phenylethynyl)-N-[(R)-2-hydroxy-1-(1H-indol-3-ylmethyl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 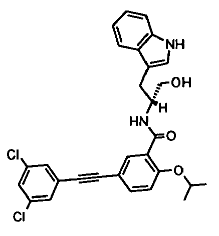 |
| | | | | |
| | *5-Ethynyl-N-[(R)-1-hydroxymethyl-2-(1H-indol-3-yl)-ethyl]-2-isopropoxy benzamide* | | | |
| | | | | |
| | *and* | | | |
| | | | | |
| | *1-Bromo-3, 5-dichloro-benzene* | | Molecular peak (ESI, M+1): 522 | |
| | | | Retention time: 5.13 min. | |
| **63** | 5-(2-Acetyl-benzofuran-5-ylethynyl)-N-[(R)-1-hydroxymethyl-2-(1H-indol-3-yl)-ethyl]-2-isopropoxy-benzamide; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 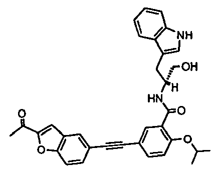 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *1-(5-Bromo-benzofuran-2-yl)-ethanone* | | Molecular peak (ESI, M+1): 536 | |
| | | | Retention time: 4.22 min. | |
| **64** | 3-Hydroxy-4-{3-[(R)-1-hydroxymethyl-2-(1H-indol-3-yl)-ethylcarbamoyl]-4-isopropoxy-phenylethynyl}-benzoic acid; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75') | 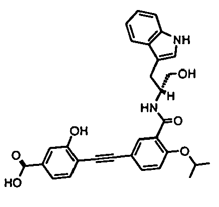 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *4-Bromo-3-hydroxy-benzoic acid* | | Molecular peak (ESI, M+1): 514 | |
| | | | Retention time: 3.72 min. | |
| **65** | 4-Hydroxy-3-{3-[(R)-1-hydroxymethyl-2-(1H-indol-3-yl)-ethylcarbamoyl]-4-isopropoxy-phenylethynyl}-benzoic acid methyl ester; | **27** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1 % to 99% (5') to 99% (1') to 1% (0.25') to 1% (1.75') | 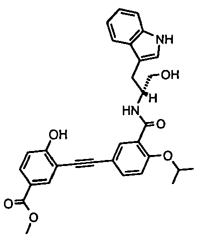 |
| | | | | |
| | *5-Ethynyl-N-[(R)-1-hydroxymethyl-2-(1H-indol-3-yl)-ethyl]-2-isopropoxy-benzamide* | | | |
| | | | | |
| | *and* | | | |
| | | | | |
| | *3-Bromo-4-hydroxy-benzoic acid methyl ester* | | Molecular peak (ESI, M+1): 528 | |
| | | | Retention time: 4.33 min. | |

## Claims

1. Compounds of the formula I 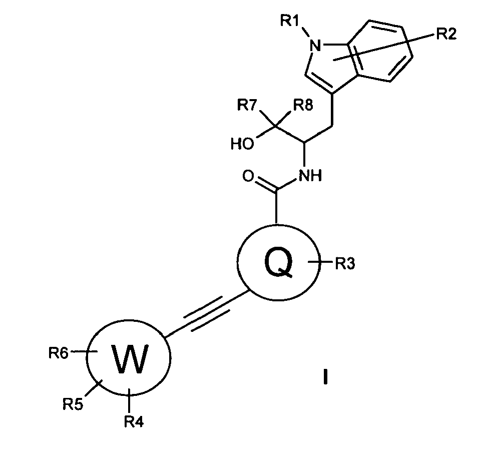 in which
R1 is hydrogen, C₁-C₆-alkyl, C₃-C₆-alkenyl, C₃-C₆-alkynyl, C₃-C₇-cycloalkyl, C₁-C₆-alkyloxy-C₁-C₆-alkylene, C₃-C₇-cycloalkyloxy-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted once or more times by fluorine, cyano, hydroxy, amino or the groups: 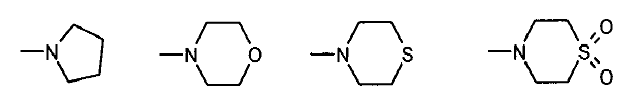 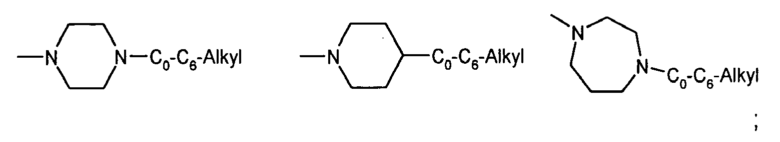
R2 is hydrogen, halogen, cyano, -SO₂Me, C₁-C₆-alkyl, C₂-C₆-alkenyl, C₂-C₆-alkynyl, C₁-C₆-alkyloxy or benzyloxy,
where the hydrocarbon chains therein may optionally be fluorinated once or more times;
R3 is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenylC₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted once or more times by fluorine, cyano, hydroxy, amino or the groups: 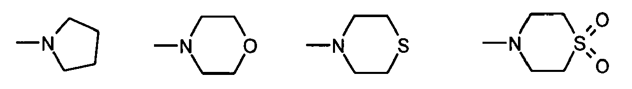 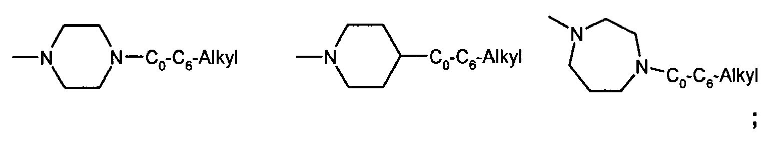
R4, R5, R6 are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or the groups: 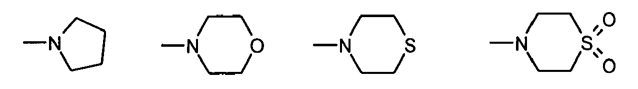 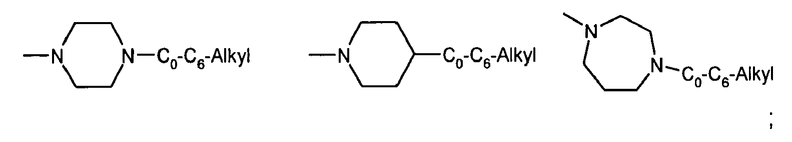 or
independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene,
C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino,
C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneaminocarbonyl, C₁-C₆-alkylcarbonyl, C₃-C₇-cycloalkylcarbonyl,
carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl,
C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)-alkyl, -N(C₀-C₆-alkyl)-C(O)-NH-C₃-C₇-cycloalkyl,
-N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl,
-C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine,
-S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine,
-O-C₂-C₆-alkylene-(C₁-C₆-alkyl)amine, -O-C₂-C₆-alkylene-[di(C₁-C₆-alkylene)]amine,
or the groups: 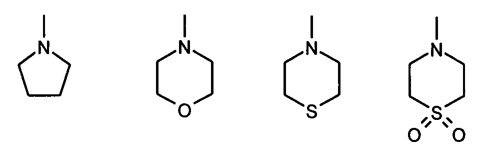 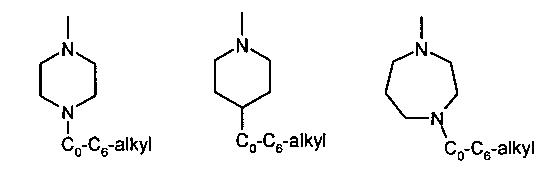 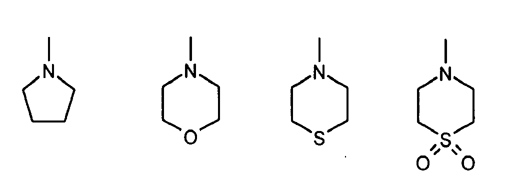 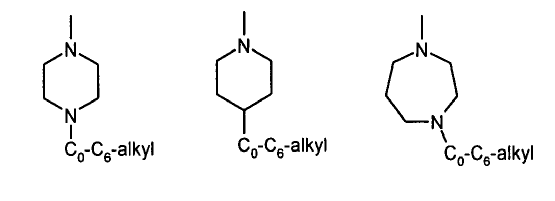 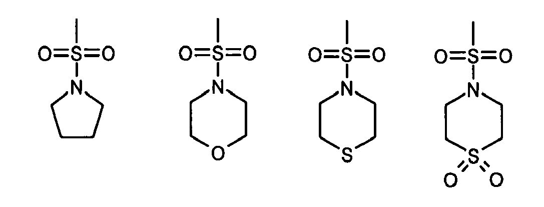 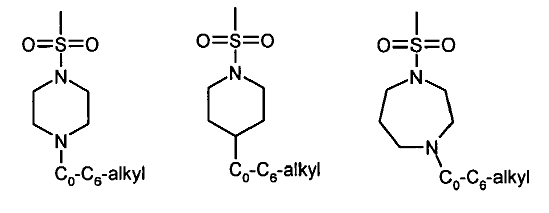 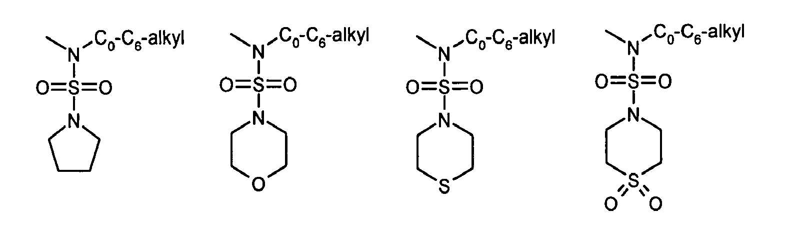 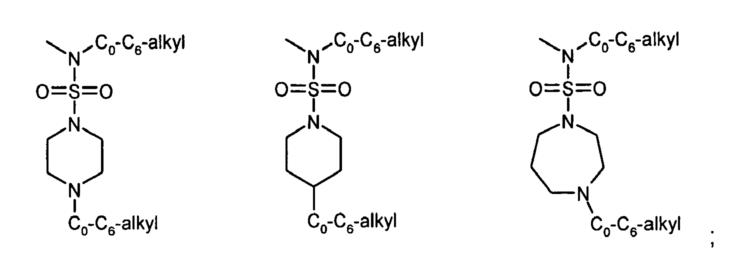
R7, R8 are independently of one another hydrogen, methyl, ethyl,
where the methyl and ethyl groups may be fluorinated once or more times;
wherein
R2 substitutes one or more positions of the aryl or heteroaryl ring in the indole residue;
R3 substitutes one or more positions of the aryl or heteroaryl ring in the group Q;
R5 and R6 may together form a heterocycloalkyl or cycloalkyl group;
Q and W are independently of one another aryl, heteroaryl.

2. Compounds according to claim 1, namely acyltryptophanols of the formula II or III 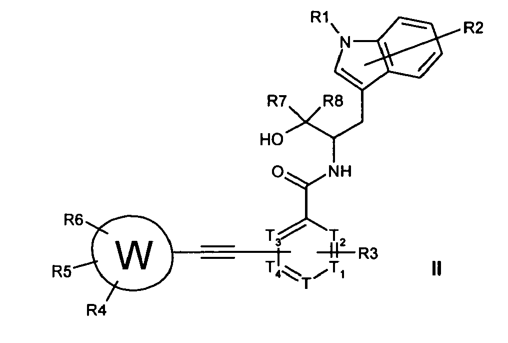 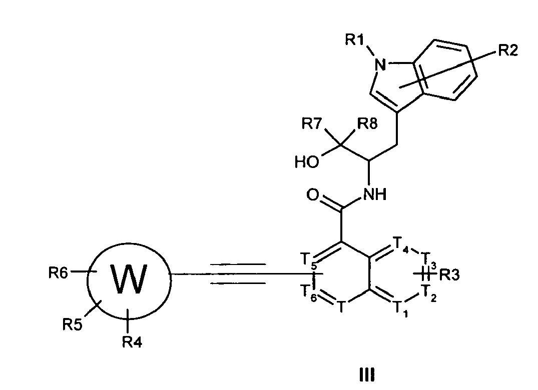 in which the groups R1 to R8 and W have the same meaning as in claim 1
and
T is a nitrogen atom or a CH group;
T1, T2, T3, T4, T5, T6 are each independently of one another a nitrogen atom or an R3-C group.

3. Compounds according to claim 1, namely acyltryptophanols of the formula IV or V 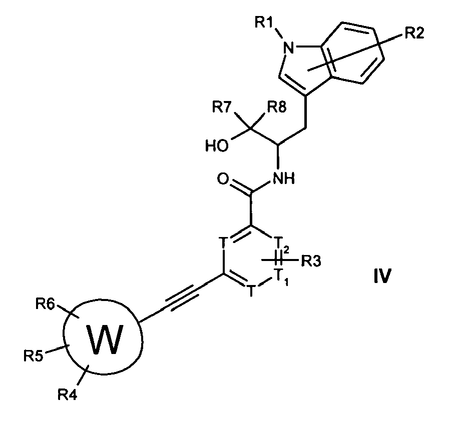 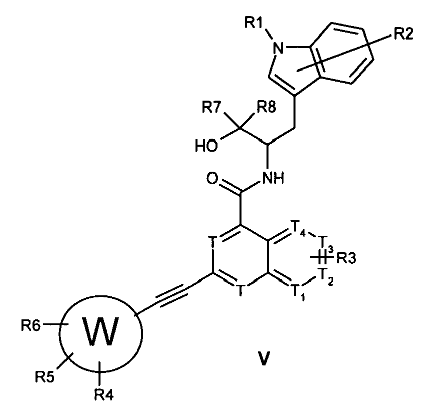 in which
R1 to R8 and W have the same meaning as in claim 1;
T is a nitrogen atom or a CH group;
T1, T2, T3, T4 are each independently of one another a nitrogen atom or an R3-C group.

4. Compounds according to any of the preceding claims, namely
**1** N-[(R)-1-Hydroxymethyl-2-(1H-indol-3-yl)-ethyl]-5-phenylethynyl-2-propoxy-benzamide
**2** N-[(R)-1-Hydroxymethyl-2-(1H-indol-3-yl)-ethyl]-5-(2-methoxy-phenylethynyl)-2-propoxy-benzamide
**3** N-[(R)-1-Hydroxymethyl-2-(1H-indol-3-yl)-ethyl]-2-isopropoxy-5-(2-methoxyphenylethynyl)-benzamide
**4** N-[(S)-1-Hydroxymethyl-2-(1H-indol-3-yl)-ethyl]-2-isopropoxy-5-(2-methoxyphenylethynyl)-benzamide
**5** N-[(R)-1-Hydroxymethyl-2-(1H-indol-3-yl)-ethyl]-2-isopropoxy-5-o-tolylethynyl-benzamide
**6** 3-(2-Methoxy-phenylethynyl)-naphthalene-1-carboxylic acid [(R)-1-hydroxymethyl-2-(1H-indol-3-yl)-ethyl]-amide
**7** 6-(2-Methoxy-phenylethynyl)-quinoline-8-carboxylic acid [(R)-1-hydroxymethyl-2-(1H-indol-3-yl)-ethyl]-amide
**8** N-[(R)-1-Hydroxymethyl-2-(1H-indol-3-yl)-ethyl]-2-isopropoxy-5-(3-ethylcarbamoylphenylethynyl)-benzamide
**9** 2-Ethoxy-N-[(R)-1-hydroxymethyl-2-(1H-indol-3-yl)-ethyl]-5-(3-methylcarbamoylphenylethynyl)-benzamide
**10** N-[(R)-1-Hydroxymethyl-2-(1H-indol-3-yl)-ethyl]-2-isopropoxy-5-(4-methylcarbamoylphenylethynyl)-benzamide
**11** N-[(R)-1-Hydroxymethyl-2-(1H-indol-3-yl)-ethyl]-2-isopropoxy-5-(1-oxo-1,2,3,4-tetrahydro-isoquinolin-7-ylethynyl)-benzamide
**12** 2-Ethoxy-N-[(R)-1-hydroxymethyl-2-(1H-indol-3-yl)-ethyl]-5-(4-methylcarbamoylphenylethynyl)-benzamide
**13** 5-(2,3-Dihydro-1H-indol-5-ylethynyl)-N-[(R)-1-hydroxymethyl-2-(1H-indol-3-yl)-ethyl]-2-isopropoxy-benzamide
**14** 5-(5-Cyanomethyl-2-methoxy-phenylethynyl)-N-[(R)-1-hydroxymethyl-2-(1H-indol-3-yl)-ethyl]-2-isopropoxy-benzamide
**15** 5-(2,3-Dimethyl-phenylethynyl)-N-[(R)-1-hydroxymethyl-2-(1H-indol-3-yl)-ethyl]-2-isopropoxy-benzamide
**16** 5-(2-Cyano-thiophen-3-ylethynyl)-N-[(R)-1-hydroxymethyl-2-(1H-indol-3-yl)-ethyl]-2-isopropoxy-benzamide
**17** N-[(R)-1-Hydroxymethyl-2-(1H-indol-3-yl)-ethyl]-2-isopropoxy-5-(2,4,6-trimethylphenylethynyl)-benzamide
**18** N-[(R)-1-Hydroxymethyl-2-(1H-indol-3-yl)-ethyl]-2-isopropoxy-5-(2,4,5-trimethylphenylethynyl)-benzamide
**19** 5-[4-(2-Hydroxy-ethyl)-phenylethynyl]-N-[(R)-1-hydroxymethyl-2-(1H-indol-3-yl)-ethyl]-2-isopropoxy-benzamide
**20** N-[(R)-2-Hydroxy-1-(1H-indol-3-ylmethyl)-2-methyl-propyl]-2-isopropoxy-5-(2-methoxyphenylethynyl)-benzamide
**21** N-[2-(5-Fluoro-1H-indol-3-yl)-1-hydroxymethyl-ethyl]-2-isopropoxy-5-phenylethynylbenzamide
**22** N-[2-(5-Fluoro-1H-indol-3-yl)-1-hydroxymethyl-ethyl]-2-isopropoxy-5-(2-methoxyphenylethynyl)-benzamide
**23** N-[(RS)-2-(5,6-Difluoro-1H-indol-3-yl)-1-hydroxymethyl-ethyl]-2-isopropoxy-5-(2-methoxy-phenylethynyl)-benzamide
**24** N-[(R)-2-(1-Ethyl-1H-indol-3-yl)-1-hydroxymethyl-ethyl]-2-isopropoxy-5-(2-methoxyphenylethynyl)-benzamide
**25** N-[(R)-2-(1-Ethyl-1H-indol-3-yl)-1-hydroxymethyl-ethyl]-2-isopropoxy-5-phenylethynylbenzamide
**26** 5-(3-Amino-1H-indazol-4-ylethynyl)-N-[(R)-2-hydroxy-1-(1H-indol-3-ylmethyl)-ethyl]-2-isopropoxy-benzamide
**27** N-[(R)-1-Hydroxymethyl-2-(1H-indol-3-yl)-ethyl]-5-(4-hydroxy-phenylethynyl)-2-isopropoxy-benzamide
**28** N-[(R)-1-Hydroxymethyl-2-(1H-indol-3-yl)-ethyl]-2-isopropoxy-5-(6-methoxynaphthalen-2-ylethynyl)-benzamide
**29** N-[(R)-1-Hydroxymethyt-2-(1H-indol-3-yl)-ethyl]-2-isopropoxy-5-m-tolyethynylbenzamide
**30** 5-(3-Fluoro-4-methoxy-phenylethynyl)-N-[(R)-2-hydroxy-1-(1H-indol-3-ylmethyl)-ethyl]-2-isopropoxy-benzamide
**31** N-[(R)-1-Hydroxymethyl-2-(1H-indol-3-yl)-ethyl]-5-(3-hydroxy-6-methyl-pyridin-2-ylethynyl)-2-isopropoxy-benzamide
**32** 4-{3-[(R)-1-Hydroxymethyl-2-(1H-indol-3-yl)-ethylcarbamoyl]-4-isopropoxyphenylethynyl}-benzoic acid methyl ester
**33** 5-{3-[(R)-1-Hydroxymethyl-2-(1H-indol-3-yl)-ethylcarbamoyl]-4-isopropoxyphenylethynyl}-nicotinic acid methyl ester
**34** 5-{3-[(R)-2-Hydroxy-1-(1H-indol-3-ylmethyl)-ethylcarbamoyl]-4-isopropoxyphenylethynyl}-thiophene-2-carboxylic acid ethyl
**35** N-[(R)-1-Hydroxymethyl-2-(1H-indol-3-yl)-ethyl]-5-(6-hydroxy-naphthalen-2-ylethynyl)-2-isopropoxy-benzamide
**36** 3-{3-[(R)-1-Hydroxymethyl-2-(1H-indol-3-yl)-ethylcarbamoyl]-4-isopropoxyphenylethynyl}-benzoic acid
**37** N-[(R)-1-Hydroxymethyl-2-(1H-indol-3-yl)-ethyl]-5-(2-hydroxymethyl-phenylethynyl)-2-isopropoxy-benzamide
**38** 5-(5-Fluoro-2-methoxy-phenylethynyl)-N-[(R)-2-hydroxy-1-(1H-indol-3-ylmethyl)-ethyl]-2-isopropoxy-benzamide
**39** 5-(3-Cyano-phenylethynyl)-N-[(R)-1-hydroxymethyl-2-(1H-indol-3-yl)-ethyl]-2-isopropoxy-benzamide
**40** 5-(3,5-Dimethyl-phenylethynyl)-N-[(R)-1-hydroxymethyl-2-(1H-indol-3-yl)-ethyl]-2-isopropoxy-benzamide
**41** 5-(2,6-Difluoro-phenylethynyl)-N-[(R)-2-hydroxy-1-(1H-indol-3-ylmethyl)-ethyl]-2-isopropoxy-benzamide
**42** 5-(3,5-Difluoro-phenylethynyl)-N-[(R)-2-hydroxy-1-(1H-indol-3-ylmethyl)-ethyl]-2-isopropoxy-benzamide
**43** N-[(R)-2-Hydroxy-1-(1H-indol-3-ylmethyl)-ethyl]-2-isopropoxy-5-(3-trifluoromethylphenylethynyl)-benzamide
**44** 5-(2-Chloro-4-hydroxy-phenylethynyl)-N-[(R)-2-hydroxy-1-(1H-indol-3-ylmethyl)-ethyl]-2-isopropoxy-benzamide
**45** N-[(R)-1-Hydroxymethyl-2-(1H-indol-3-yl)-ethyl]-2-isopropoxy-5-(4-methoxyphenylethynyl)-benzamide
**46** N-[(R)-1-Hydroxymethyl-2-(1H-indol-3-yl)-ethyl]-2-isopropoxy-5-pyrimidin-5-ylethynylbenzamide
**47** N-[(R)-1-Hydroxymethyl-2-(1H-indol-3-yl)-ethyl]-5-(3-hydroxy-phenylethynyl)-2-isopropoxy-benzamide
**48** 5-(2,5-Dimethyl-phenylethynyl)-N-[(R)-1-hydroxymethyl-2-(1H-indol-3-yl)-ethyl]-2-isopropoxy-benzamide
**49** 5-(3,4-Dichloro-phenylethynyl)-N-[(R)-2-hydroxy-1-(1H-indol-3-ylmethyl)-ethyl]-2-isopropoxy-benzamide
**50** 5-Benzo[1,3]dioxol-5-ylethynyl-N-[(R)-1-hydroxymethyl-2-(1H-indol-3-yl)-ethyl]-2-isopropoxy-benzamide
**51** 5-(2-Cyano-phenylethynyl)-N-[(R)-1-hydroxymethyl-2-(1H-indol-3-yl)-ethyl]-2-isopropoxy-benzamide
**52** N-[(R)-1-Hydroxymethyl-2-(1H-indol-3-yl)-ethyl]-2-isopropoxy-5-naphthalen-2-ylethynyl-benzamide
**53** 5-(4-Fluoro-2-methyl-phenylethynyl)-N-[(R)-2-hydroxy-1-(1H-indol-3-ylmethyl)-ethyl]-2-isopropoxy-benzamide
**54** N-[(R)-1-Hydroxymethyl-2-(1H-indol-3-yl)-ethyl]-2-isopropoxy-5-naphthalen-1-ylethynyl-benzamide
**55** 5-(2,4-Dimethyl-phenylethynyl)-N-[(R)-1-hydroxymethyl-2-(1H-indol-3-yl)-ethyl]-2-isopropoxy-benzamide
**56** 5-(2-Fluoro-phenylethynyl)-N-[(R)-2-hydroxy-1-(1H-indol-3-ylmethyl)-ethyl]-2-isopropoxy-benzamide
**57** N-[(R)-1-Hydroxymethyl-2-(1H-indol-3-yl)-ethyl]-2-isopropoxy-5-(3-methoxyphenylethynyl)-benzamide
**58** (2-{3-[(R)-1-Hydroxymethyl-2-(1H-indol-3-yl)-ethylcarbamoyl]-4-isopropoxyphenylethynyl}-phenyl)-acetic acid
**59** 5-(4-Hydroxy-biphenyl-3-ylethynyl)-N-[(R)-1-hydroxymethyl-2-(1H-indol-3-yl)-ethyl]-2-isopropoxy-benzamide
**60** 5-(3-Chloro-4-cyano-phenylethynyl)-N-[(R)-2-hydroxy-1-(1H-indol-3-ylmethyl)-ethyl]-2-isopropoxy-benzamide
**61** 5-(3-Acetyl-phenylethynyl)-N-[(R)-1-hydroxymethyl-2-(1H-indol-3-yl)-ethyl]-2-isopropoxy-benzamide
**62** 5-(3,5-Dichloro-phenylethynyl)-N-[(R)-2-hydroxy-1-(1H-indol-3-ylmethyl)-ethyl]-2-isopropoxy-benzamide
**63** 5-(2-Acetyl-benzofuran-5-ylethynyl)-N-[(R)-1-hydroxymethyl-2-(1H-indol-3-yl)-ethyl]-2-isopropoxy-benzamide
**64** 3-Hydroxy-4-{3-[(R)-1-hydroxymethyl-2-(1H-indol-3-yl)-ethylcarbamoyl]-4-isopropoxyphenylethynyl}-benzoic acid
**65** 4-Hydroxy-3-{3-[(R)-1-hydroxymethyl-2-(1H-indol-3-yl)-ethylcarbamoyl]-4-isopropoxyphenylethynyl}-benzoic acid methyl ester

5. Process for preparing compounds of the formula I of claim 1, wherein a tryptophanol derivative of the formula VI 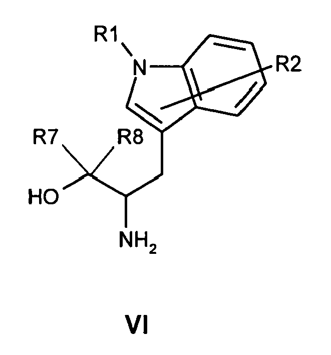 is coupled with a carboxylic acid of the formula VII 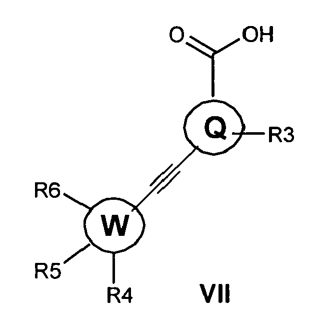 in an amide forming reaction comprising
a) conversion of said carboxylic acids into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said tryptophanol.

6. Process according to claim 5 for preparing compounds of the formulae II or III of claim 2, wherein a tryptophanol derivative of the formula VI is coupled with a carboxylic acid of the formulae VIII or IX

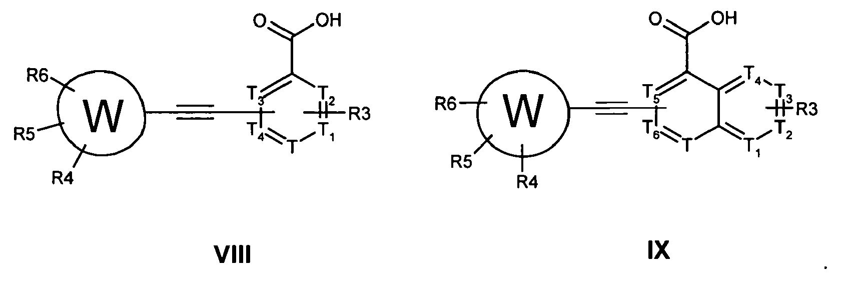

7. Process according to claim 5 for preparing compounds of the formulae IV or V of claim 3, wherein a tryptophanol derivative of the formula VI is coupled with a carboxylic acid of the formulae X or XI

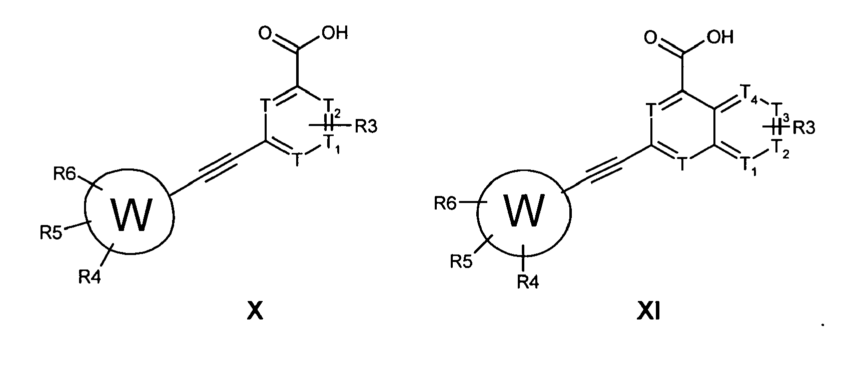

8. Process for preparing compounds of the formula I of claim 1, wherein an aryl halide of the formula XII 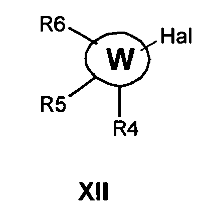 is coupled with an acetylene of the formula XIII 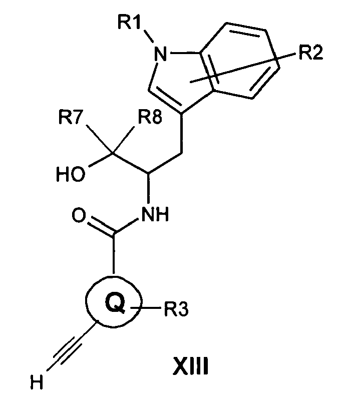 in the presence of a palladium catalyst, and optionally a Cu (I) source, and optionally a base
in a Sonogashira type reaction.

9. Process according to claim 8 for preparing compounds of the formula II of claim 2, wherein an aryl halide of formula XII is coupled with an acetylene of the formula XVI

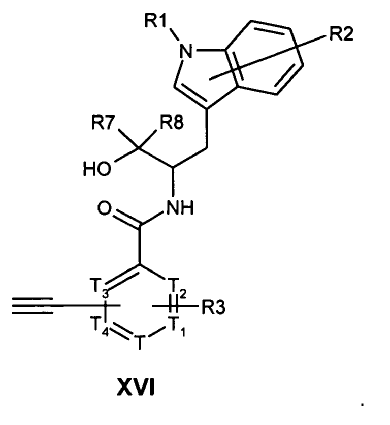

10. Process according to claim 8 for preparing compounds of the formula III of claim 2, wherein an aryl halide of formula XII is coupled with an acetylene of the formula XVIII

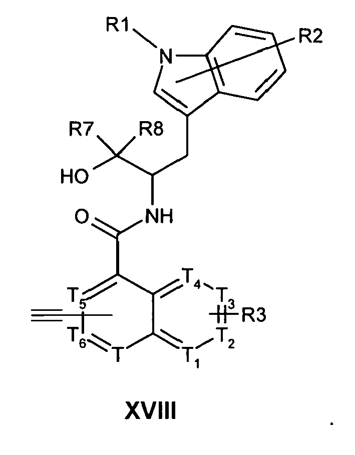

11. Process according to claim 8 for preparing compounds of the formula IV of claim 3, wherein an aryl halide of formula XII is coupled with an acetylene of the formula XX

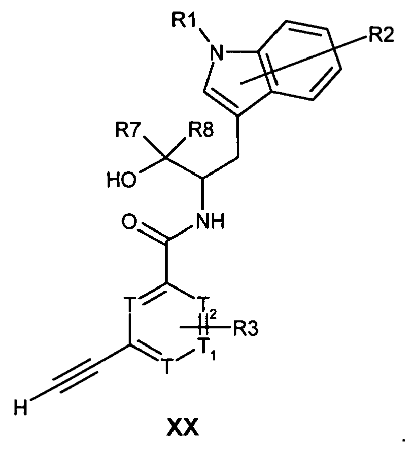

12. Process according to claim 8 for preparing compounds of the formula V of claim 3, wherein an aryl halide of formula XII is coupled to an acetylene of the formula XXII

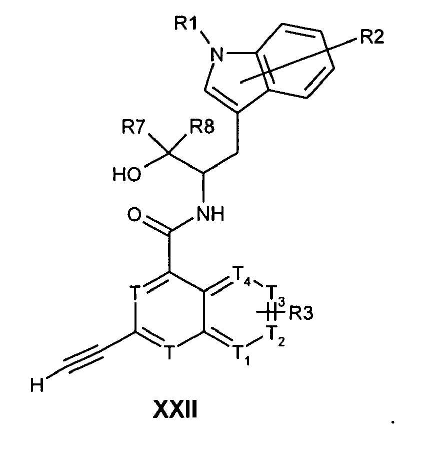

13. Process for preparing compounds of the formula I of claim 1, wherein an acetylene of th formula XIV 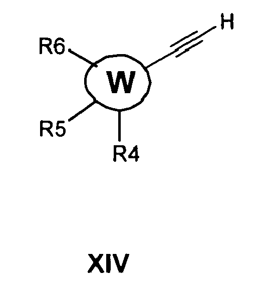 is coupled with an aryl halide of the formula XV 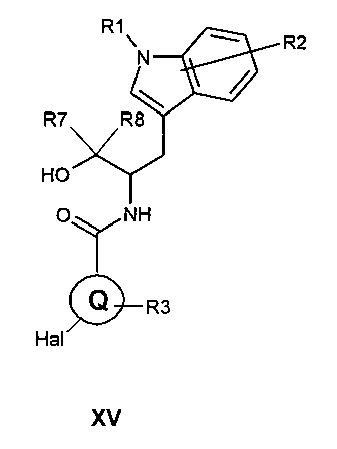 in the presence of a palladium catalyst, and optionally a Cu (I) source, and optionally a base
in a Sonogashira type reaction.

14. Process according to claim 13 for preparing compounds of the formula II of claim 2, wherein an acetylene of formula XIV is coupled with an aryl halide of the formula XVII

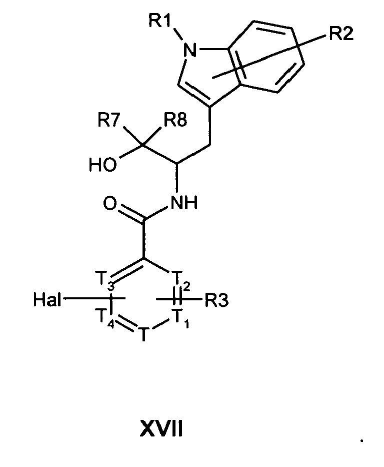

15. Process according to claim 13 for preparing compounds of the formula III of claim 2, wherein an acetylene of formula XIV is coupled with an aryl halide of the formula XIX

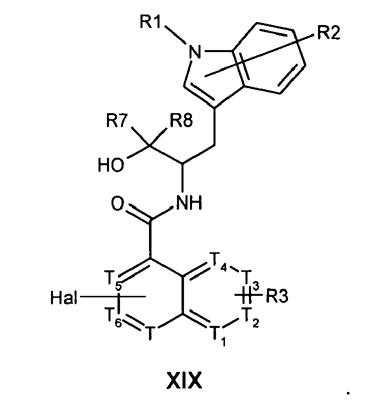

16. Process according to claim 13 for preparing compounds of the formula IV of claim 3, wherein an acetylene of the formula XIV is coupled with an aryl halide of the formula XXI

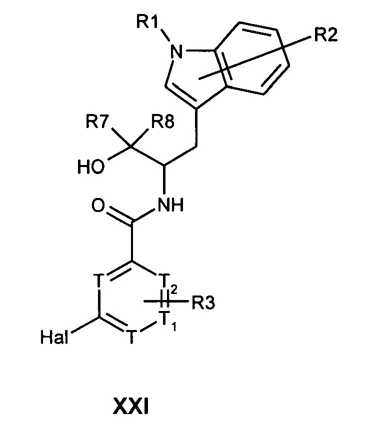

17. Process according to claim 13 for preparing compounds of the formula V of claim 3, wherein an acetylene of the formula XIV is coupled with an aryl halide of the formula XXIII

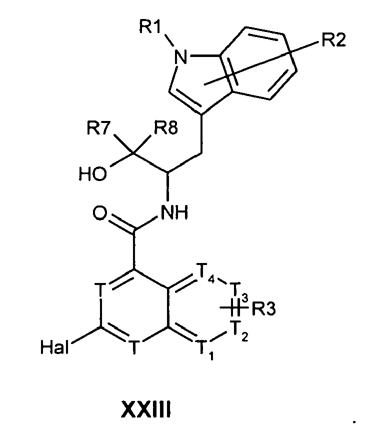

18. Carboxylic acids as intermediates in a process according to any of claims 5 to 7, namely
5-Phenylethynyl-2-propoxy-benzoic acid;
5-(2-Methoxy-phenylethynyl)-2-propoxy-benzoic acid;
2-Isopropoxy-5-(2-methoxy-phenylethynyl)-benzoic acid
and their methyl, ethyl, propyl and butyl esters.

19. Acetylene derivatives as intermediates in a process according to any of claims 8 to 17, namely
5-Ethynyl-N-[(R)-1-hydroxymethyl-2-(1H-indol-3-yl)-ethyl]-2-isopropoxybenzamide;
2-Ethoxy-5-ethynyl-N-[(R)-1-hydroxymethyl-2-(1H-indol-3-yl)-ethyl]-benzamide.

20. Aryl halides as intermediates in a process according to any of claims 8 to 17, namely
3-Bromo-naphthalene-1-carboxylic acid [(R)-1-hydroxymethyl-2-(1H-indol-3-yl)-ethyl]-amide;
6-Bromo-quinoline-8-carboxylic acid [(R)-1-hydroxymethyl-2-(1H-indol-3-yl)-ethyl]-amide.

21. Pharmaceutical compositions comprising at least one of the compounds according to any of claims 1 to 4 with pharmacologically suitable recipients and carriers.

22. Use of the compounds of the general formula I according to any of Claims 1 to 4 for fertility control in men or in women.

23. Process for producing medicaments comprising at least one of the compounds of the general formula I according to any of Claims 1 to 4 for the prevention and/or treatment of osteoporosis.
